# EUROPEAN PATENT SPECIFICATION

(11) **EP 4 232 122 B1**
(45) Date of publication and mention of the grant of the patent: **26.08.2026**
(21) Application number: 21807375.7
(22) Date of filing: 19.10.2021
(51) Int. Cl.: A61M 5/20, A61M 5/315

(54) **MEDICATION DELIVERY DEVICE WITH MOISTURE SENSING SYSTEM**
ARZNEIMITTELABGABEVORRICHTUNG MIT FEUCHTIGKEITSSENSORSYSTEM
DISPOSITIF D'ADMINISTRATION DE MÉDICAMENTS AVEC SYSTÈME DE DÉTECTION DE L'HUMIDITÉ

(30) Priority: 22.10.2020 US 202063094970 P
(43) Date of publication of application: 30.08.2023
(60) Divisional application: 26189209.5 / 4 792 860
(73) Proprietor: Eli Lilly and Company, Indianapolis, IN 46285 (US)
(72) Inventor: BOWYER, Andrew Eric, Indianapolis, Indiana 46206-6288 (US); TRZYBINSKI, Robert Eugene, Indianapolis, Indiana 46206-6288 (US); ASH, Matthew James, Indianapolis, Indiana 46206-6288 (US); PREDA, Emil, Indianapolis, Indiana 46206-6288 (US)
(74) Representative: J A Kemp LLP
(86) International application number: PCT/US2021/055492
(87) International publication number: WO 2022/086885

(56) References cited:
- WO-A1-2013/022742
- WO-A1-2020/176319
- AU-B2- 2013 206 130
- AU-B2- 2018 319 545
- US-A1- 2020 206 431
- US-B1- 9 636 464
- US-B2- 9 335 294

## Description

### BACKGROUND

Patients suffering from various diseases must frequently inject themselves with medication. To allow a person to conveniently and accurately self-administer medicine, a variety of devices broadly known as pen injectors or injection pens have been developed. Generally, these pens are equipped with a cartridge including a piston and containing a multi-dose quantity of liquid medication. A drive member is movable forward to advance the piston in the cartridge to dispense the contained medication from an outlet at the distal cartridge end, typically through a needle.

In disposable or prefilled pens, after a pen has been utilized to exhaust the supply of medication within the cartridge, a user discards the entire pen and begins using a new replacement pen. In reusable pens, after a pen has been utilized to exhaust the supply of medication within the cartridge, the pen is disassembled to allow replacement of the spent cartridge with a fresh cartridge, and then the pen is reassembled for its subsequent use.

Such devices may have components that physically interact with one another to result in a state change or an action by the device. For example, the device may have a cap that is removed prior to delivery, a dose button that may be rotated to set a dose and/or actuated to deliver a dose, an "on" button that wakes the device, and so on.

Such devices can include electronics, such as an integrated circuit with a processing unit and other components. For example, the electronics can include a sensing device, such as a switch, in communication with a processing unit to detect the occurrence of such interactions. The electronics may not be within a water-tight enclosure. The inventors have appreciated that the electronics can be exposed to moisture, which can affect the operation of the electronics. In particular, the inventors have appreciated that moisture can cause the electronics to record, process, and/or store erroneous data regarding such interactions. The inventors have thus recognized a need for a moisture sensing mechanism that can be used to sense moisture on the electronics and prevent erroneous data from being used by the system.

US 2020/206431 A1 discloses a dose detection system for use with a medication delivery device in which a dose setting member rotates relative to an actuator during dose delivery. The dose detection system comprises a module including a rotation sensor attached to the actuator during dose delivery.

WO 2020/176319 A1 discloses medication delivery devices having a housing comprising a reservoir sized sufficiently to hold medication, a dose button being rotatable relative to the housing to select a dose size of the medication for an injection, a printed circuit board, a switch mounted to the printed circuit board, and a controller in communication with the switch.

WO 2013/022742 A1 discloses a wetness sensor that includes a self-supporting substrate and an electrically conductive trace carried by the substrate. The trace is patterned to provide at least a portion of a tuned RF circuit, which may be disposed on only one side of the substrate and characterized by an impedance or resistance. The trace is not self-supporting. The substrate is adapted to dissolve, swell, or otherwise degrade when contacted by a target fluid. Such degradation produces a drastic change in the operation of the RF circuit, which can be interpreted by a remote reader as a "wet" condition.

US 9 636 464 B1 discloses a drug delivery device which includes a first housing part, a second housing part, a button, a drug expelling mechanism, a graduated plate, a circuit board and a pressing rod. The button is connected to the first housing part. The drug expelling mechanism is disposed inside the second housing part, and includes a dose setting member which is rotatable and configured to set a drug dose to be expelled. The graduated plate is disposed inside the first housing part and connected to the button. The circuit board is disposed inside the first housing part and connected to the dose setting member. The pressing rod is inserted through the graduated plate, and includes an end connected to the button and the other end movable on an axial direction to trigger an actuating switch.

### SUMMARY

The scope of the invention is defined in the appended claims. Any "aspect", "example" and "embodiment" of the description not falling within the scope of the claims does not form part of the invention and is provided for illustrative purposes only. The present disclosure relates to a medication delivery device that includes circuitry and/or logic to monitor for the presence of moisture on a printed circuit board of the medication delivery device. According to some embodiments, the techniques can use existing circuitry of the medication delivery device (e.g., bias sources, analog-to-digital converters, and/or the like) that are not traditionally used to sense for moisture.

In one embodiment, a medication delivery device includes: a housing comprising a reservoir sized sufficiently to hold medication; a dose button being rotatable relative to the housing to select a dose size of the medication for an injection; a printed circuit board; a conductive trace disposed at least partially on the printed circuit board; a bias source in electrical communication with the conductive trace; and a microcontroller in electrical communication with the conductive trace through a logic input to the microcontroller, the microcontroller being configured to: receive a signal from the conductive trace through the logic input; and determine, based on the received signal, that moisture may be present on the printed circuit board.

In one embodiment, a medication delivery device includes: a housing comprising a reservoir sized sufficiently to hold medication; a dose button being rotatable relative to the housing to select a dose size of the medication for an injection; a printed circuit board; a conductive trace disposed at least partially on the printed circuit board; an analog-to-digital converter (ADC) comprising an input and an output, wherein: the input of the ADC is in electrical communication with the conductive trace; and an operating input range of the ADC is between a low input voltage and a high input voltage; a bias source in electrical communication with the input of the ADC, wherein the bias source is configured to provide a bias voltage between the low input voltage and the high input voltage; and a microcontroller in electrical communication with the output of the ADC, the microcontroller being configured to: receive a signal from the output of the ADC; and determine, based on the received signal, that moisture may be present on the printed circuit board.

### BRIEF DESCRIPTION OF THE DRAWINGS

Additional embodiments of the disclosure, as well as features and advantages thereof, will become more apparent by reference to the description herein taken in conjunction with the accompanying drawings. The components in the figures are not necessarily to scale. Moreover, in the figures, like-referenced numerals designate corresponding parts throughout the different views.
FIG. 1 is a perspective view of a medication delivery device having a dose detection system according to aspects of the present disclosure.
FIG. 2 is a partially exploded perspective view of the medication delivery device of FIG. 1, showing a dose button having a support and a cover, where the cover is shown separated from the support.
FIG. 3 is a partially exploded perspective view of the medication delivery device of FIG. 1 showing the components of the dose detection system.
FIG. 4 is a cross-sectional view of the medication delivery device of FIG. 1.
FIG. 5 is a partial cutaway view of a proximal end of the medication delivery device of FIG. 1, showing components of the dose detection system.
FIG. 6 is an underside view of a portion of the dose button of FIG. 1, showing a printed circuit board held within the dose button cover.
FIG. 7 is an exploded view of the portion of the dose button shown in FIG. 6.
FIG. 8 is a perspective view of a flange of a dose detection system of a medication delivery device.
FIG. 9 is a top down view of the flange of FIG. 8.
FIG. 10 is a perspective view of a dose button support.
FIG. 11 is a top down view of the dose button support of FIG. 10.
FIG. 12 is a perspective view of a printed circuit board and sensor switch according to aspects of the present disclosure.
FIG. 13 is a perspective view of a cantilevered arm and base of the sensor switch of FIG. 12.
FIG. 14 is a side view of the cantilevered arm and base of FIG. 13.
FIG. 15 is a side view of the cantilevered arm of FIG. 12 positioned between two teeth of a flange.
FIG. 16 shows the cantilevered arm of FIG. 15 being pushed by one of the teeth of the flange during rotation of the flange.
FIG. 17 shows the cantilevered arm being pushed further by the tooth of the flange such that a portion of the cantilevered arm has moved toward and is in contact with a conductive pad, closing the switch.
FIG. 18 shows the cantilevered arm sliding over the tooth of the flange.
FIG. 19 shows the cantilevered arm interacting with the next adjacent tooth of the flange.
FIG. 20 is an exemplary schematic diagram of a printed circuit board, according to some embodiments.
FIG. 21 shows an example of a printed circuit board with a bias source, according to some embodiments.
FIG. 22 is a flow chart showing a first exemplary computerized method that can be executed by the microcontroller of a medication delivery device to determine whether there is likely moisture present in the medication delivery device, according to some embodiments.
FIG. 23 shows an example of a printed circuit board with a bias source and an ADC, according to some embodiments.
FIG. 24 is a flow chart showing a first exemplary computerized method that can be executed by the microcontroller of a medication delivery device to determine whether there is likely moisture present in the medication delivery device, according to some embodiments.

### DETAILED DESCRIPTION

For the purposes of promoting an understanding of the principles of the present disclosure, reference will now be made to the embodiments illustrated in the drawings, and specific language will be used to describe the same. It will nevertheless be understood that no limitation of the scope of the invention is thereby intended.

The present disclosure relates to moisture sensing systems for medication delivery devices. The presence of moisture on the printed circuit board of a medication delivery device can cause errors. In particular, the moisture can electrically connect components on the board (e.g., voltage sources, test pads, ground, etc.) that are not normally electrically connected, which can cause various errors. For example, the presence of moisture can negatively impact the operation of the medication delivery device, such as causing the medication delivery device to detect incorrect information (e.g., such as incorrectly detecting the occurrence of an event, when the event did not occur) and/or to detect incorrect data (e.g., detecting erroneous data for an injection event). It is therefore desirable to detect the potential presence of moisture, and to modify the operation of the medication delivery device accordingly (e.g., to avoid erroneous data from being saved and/or reported).

In some embodiments, the sensing system includes circuitry and/or logic that detects the presence of potential moisture on a printed circuit board of the medication delivery device. In some cases, physical space for housing such circuitry and/or logic on such a printed circuit board may be limited for various reasons, e.g., because there is a desire to keep the form factor of such medication delivery devices small, or to prevent or mitigate disruptions to manufacturing processes of existing medication delivery devices. Also in some cases, there may be a need to limit the additional manufacturing cost or complexity of acquiring or integrating such moisture-detecting circuitry and/or logic. As such, the inventors have appreciated that adding additional components specifically engineered and/or dedicated to detect moisture may, in some cases, be undesirable. Rather, the inventors have appreciated that traditional circuit-based components, such as printed circuit board traces, bias sources (e.g., resistors), logic inputs (e.g., general-purpose input/outputs), and/or analog-to-digital converters, which are not traditionally used for moisture sensing, can be adapted according to the techniques described herein to sense for the potential presence of moisture.

In one aspect, the medication delivery device includes a housing that has a reservoir sized sufficiently to hold medication (e.g., insulin), and a dose button being rotatable relative to the housing to select a dose size of the medication for an injection. The medication delivery device also includes a printed circuit board, a conductive trace disposed at least partially on the printed circuit board, a bias source in electrical communication with the conductive trace, and a microcontroller in electrical communication with the conductive trace through a logic input to the microcontroller. The microcontroller is configured to receive a signal from the conductive trace through the logic input, and determine, based on the received signal, that moisture may be present on the printed circuit board.

In one aspect, the medication delivery device includes a housing comprising a reservoir sized sufficiently to hold medication, and a dose button being rotatable relative to the housing to select a dose size of the medication for an injection. The medication delivery device also includes a printed circuit board, a conductive trace disposed at least partially on the printed circuit board, and an analog-to-digital converter (ADC) comprising an input and an output. The input of the ADC is in electrical communication with the conductive trace, and an operating input range of the ADC is between a low input voltage and a high input voltage. The medication delivery device also includes a bias source in electrical communication with the input of the ADC, wherein the bias source is configured to provide a bias voltage between the low input voltage and the high input voltage, and a microcontroller in electrical communication with the output of the ADC. The microcontroller is configured to receive a signal from the output of the ADC, and determine, based on the received signal, that moisture may be present on the printed circuit board.

Devices described herein may comprise a medication, such as for example, within a reservoir or cartridge 20 (described below). In another embodiment, a system may comprise one or more devices including device 10 (described below) and a medication. The term "medication" refers to one or more therapeutic agents including but not limited to insulins, insulin analogs such as insulin lispro or insulin glargine, insulin derivatives, GLP-1 receptor agonists such as dulaglutide or liraglutide , glucagon, glucagon analogs, glucagon derivatives, gastric inhibitory polypeptide (GIP), GIP analogs, GIP derivatives, oxyntomodulin analogs, oxyntomodulin derivatives, therapeutic antibodies and any therapeutic agent that is capable of delivery by the devices described herein. The medication as used in the device may be formulated with one or more excipients. The device is operated in a manner generally as described above by a patient, caregiver or healthcare professional to deliver medication to a person.

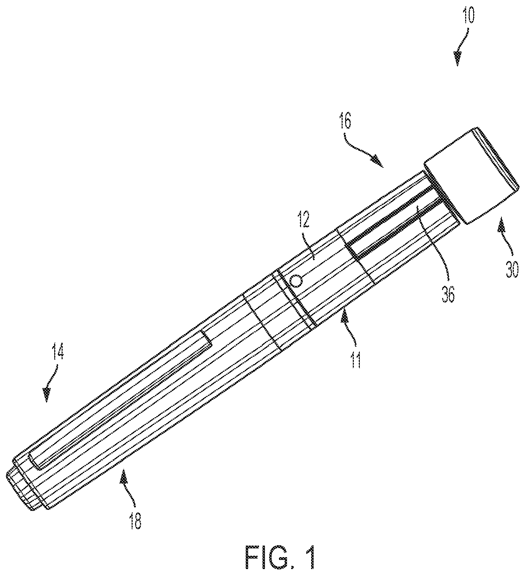

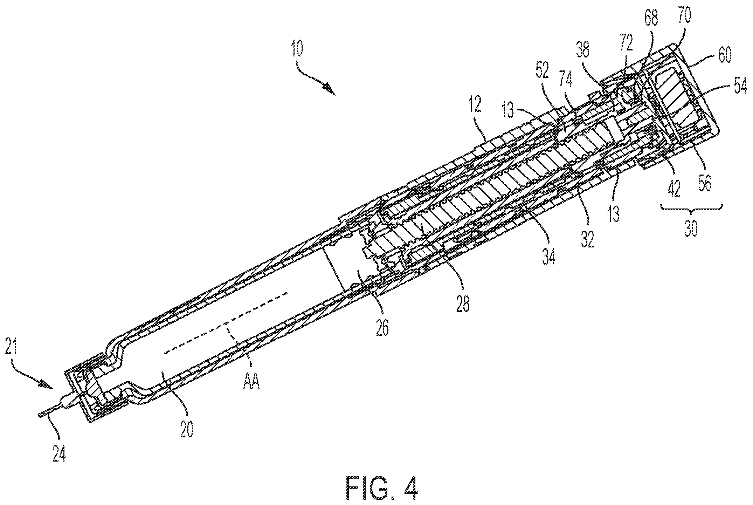

An exemplary medication delivery device 10 is illustrated in FIGS. 1-4 as a pen injector configured to inject a medication into a patient through a needle. Device 10 includes a body 11 that may comprise an elongated, pen-shaped housing 12 including a distal portion 14 and a proximal portion 16. As used herein, the term "distal" refers to the direction and/or portion of a medication delivery device that is pointed towards (or located closer to) the site of injection, while the term "proximal" refers to the direction and/or portion of a medication delivery device that is pointed away from (or located further away from) the site of injection. Distal portion 14 may be received within a pen cap 18. Referring to FIG. 4, distal portion 14 may contain a reservoir or cartridge 20 configured to hold medication to be dispensed through the outlet 21 of the housing a dispensing operation. The outlet 21 of distal portion 14 may be equipped with an injection needle 24. In some embodiments, the injection needle is removable from the housing. In some embodiments, the injection needle is replaced with a new injection needle after each use.

A piston 26 may be positioned in reservoir 20. The medication delivery device may include an injecting mechanism positioned in proximal portion 16 that is operative to advance piston 26 toward the outlet of reservoir 20 during the dose dispensing operation to force the contained medicine through the needled end. The injecting mechanism may include a drive member 28, illustratively in the form of a screw, that is axially moveable relative to housing 12 to advance piston 26 through reservoir 20.

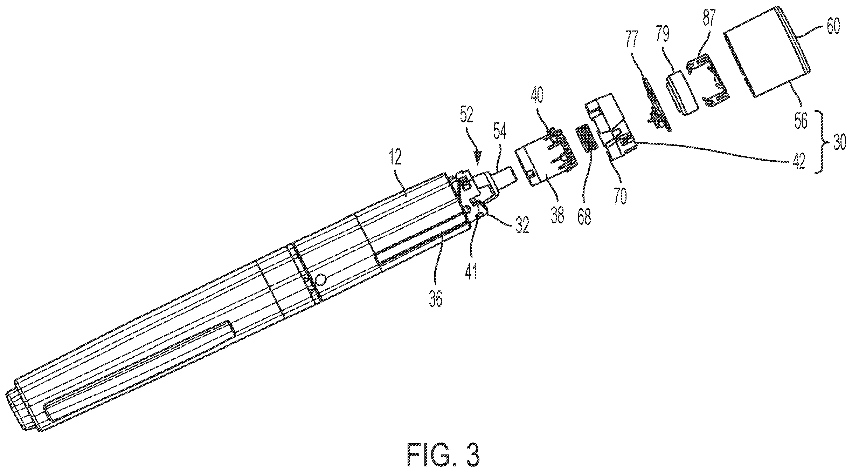

The device may include a dose-setting assembly coupled to the housing 12 for setting a dose amount to be dispensed by device 10. As best seen in FIGS. 3 and 4, in the illustrated embodiment, the dose-setting assembly includes a dose-setting screw 32 and a flange 38. The dose-setting screw 32 is in the form of a screw element operative to spiral (i.e., simultaneously move axially and rotationally) about a longitudinal axis AA of rotation relative to housing 12 during dose setting and dose dispensing. FIGS. 3 and 4 illustrate the dose-setting screw 32 fully screwed into housing 12 at its home or zero dose position. Dose-setting screw 32 is operative to screw out in a proximal direction from housing 12 until it reaches a fully extended position corresponding to a maximum dose deliverable by device 10 in a single injection. The extended position may be any position between a position corresponding to an incremental extended position (such as a dose setting a 0.5 or 1 unit) to a fully extended position corresponding to a maximum dose deliverable by device 10 in a single injection and to screw into housing 12 in a distal direction until it reaches the home or zero position corresponding to a minimum dose deliverable by device 10 in a single injection.

Referring to FIGS. 3 and 4, dose-setting screw 32 includes a helically threaded outer surface that engages a corresponding threaded inner surface 13 of housing 12 to allow dose-setting screw 32 to spiral (i.e. simultaneously rotate and translate) relative to housing 12. Dose-setting screw 32 further includes a helically threaded inner surface that engages a threaded outer surface of sleeve 34 (FIG. 4) of device 10. The outer surface of dose-setting screw 32 includes dose indicator markings, such as numbers that are visible through a dosage window 36 to indicate to the user the set dose amount.

As mentioned above, in some embodiments, the dose-setting assembly further includes a tubular flange 38 that is coupled in the open proximal end of dose-setting screw 32 and is axially and rotationally locked to the dose-setting screw 32 by protrusions 40 received within openings 41 in the dose-setting screw 32. The protrusions 40 of the flange 38 can be seen in FIGS. 3, 8 and 9, and the openings 41 of the dose-setting screw 32 can be seen in FIG. 3.

As seen in FIGS. 3 and 4, delivery device 10 may include an actuator assembly having a clutch 52 and a dose button 30. The clutch 52 is received within the dose-setting screw 32, and the clutch 52 includes an axially extending stem 54 at its proximal end. The dose button 30 of the actuator assembly is positioned proximally of the dose-setting screw 32 and flange 38. Dose button 30 includes a support 42, also referred to herein as an "under button," and a cover 56, also referred to herein as an "over button." As will be discussed, the support 42 and cover 56 enclose electronics components used to store and/or communicate data relating to amount of dose delivered by a medication delivery device.

The support 42 of the dose button may be attached to the stem 54 of the clutch 52, such as with an interference fit or an ultrasonic weld, so as to axially and rotatably fix together dose button 30 and clutch 52.

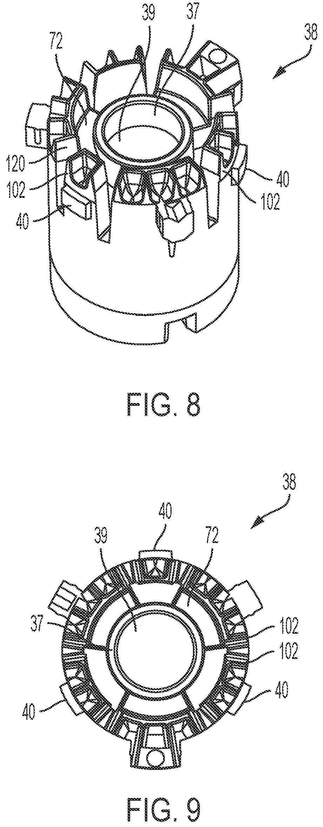

In some embodiments, a portion of the clutch may pass through a lumen 39 of the flange 38. The lumen 39 of the flange is best seen in FIGS. 8 and 9. The lumen 39 may, in some embodiments, serve to help center the clutch 52 in place.

Proximal face 60 of the dose button 30 may serve as a push surface against which a force can be applied manually, i.e., directly by the user to push the actuator assembly (dose button 30 and clutch 52) in a distal direction. A bias member 68, illustratively a spring, may be disposed between the distal surface 70 of support 42 and a proximal surface 72 of tubular flange 38 (FIGS. 8 and 9) to urge the support 42 of the actuation assembly and the flange 38 of the dose-setting assembly axially away from each other. Dose button 30 is depressible by a user to initiate the dose dispensing operation. In some embodiments, the bias member 68 is seated against this proximal surface 72 and may surround a raised collar 37 of the flange 38.

Delivery device 10 is operable in a dose setting mode and a dose dispensing mode. In the dose setting mode of operation, the dose button 30 is rotated relative to housing 12 to set a desired dose to be delivered by device 10. In some embodiments, rotating the dose button 30 in one direction relative to the housing 12 causes the dose button 30 to axially translate proximally relative to the housing 12, and rotating the dose button 30 in the opposite direction relative to the housing 12 causes the dose button 30 to axially translate distally relative to the housing. In some embodiments, clockwise rotation of the dose button moves the dose button 30 distally, and counter-clockwise rotation of the dose button moves the dose button proximally, or vice versa.

In some embodiments, rotating the dose button 30 to axially translate the dose button 30 in the proximal direction serves to increase the set dose, and rotating the dose button 30 to axially translate the dose button 30 in the distal direction serves to decrease the set dose. The dose button 30 is adjustable in pre-defined rotational increments corresponding to the minimum incremental increase or decrease of the set dose during the dose setting operation. The dose button may include a detent mechanism such that each rotational increment produces an audible and/or tactile "click." For example, one increment or "click" may equal one-half or one unit of medication.

In some embodiments, the set dose amount may be visible to the user via the dial indicator markings shown through a dosage window 36. During the dose setting mode, the actuator assembly, which includes the dose button 30 and clutch 52, moves axially and rotationally with the dose-setting assembly, which includes the flange 38 and the dose-setting screw 32.

Dose-setting screw 32 and flange 38 are fixed rotationally to one another, and rotate and move proximally during dose setting, due to the threaded connection of the dose-setting screw 32 with housing 12. During this dose setting motion, the dose button 30 is rotationally fixed relative to the flange 38 and the dose-setting screw 32 by complementary splines 74 of flange 38 and clutch 52 (FIG. 4), which are urged together by the bias member 68. In the course of dose setting, the dose-setting screw 32, flange 38, clutch 52, and dose button 30 move relative to the housing 12 in a spiral manner (i.e. simultaneous rotation and axial translation) from a "start" position to an "end" position. This rotation and translation relative to the housing is in proportion to the amount of dose set by operation of the medication delivery device 10.

Once the desired dose is set, device 10 is manipulated so the injection needle 24 properly penetrates, for example, a user's skin. The dose dispensing mode of operation is initiated in response to an axial distal force applied to the proximal face 60 of dose button 30. The axial force is applied by the user directly to dose button 30. This causes axial movement of the actuator assembly (dose button 30 and clutch 52) in the distal direction relative to housing 12.

The axial shifting motion of the actuator assembly compresses biasing member 68 and reduces or closes the gap between dose button 30 and the tubular flange 38. This relative axial movement separates the complementary splines 74 on clutch 52 and flange 38, and thereby disengages the dose button 30 from being rotationally fixed to the flange 38 and the dose-setting screw 32. In particular, the dose-setting screw 32 is rotationally uncoupled from the dose button 30 to allow backdriving rotation of the dose-setting screw 32 relative to the dose button 30 and the housing 12. Also, while the dose-setting screw 32 and flange 38 are free to rotate relative to the housing 12, the dose button 30 is held from rotating relative to the housing 12 by the user's engagement of dose button 30 by pressing against it.

As dose button 30 and clutch 52 are continued to be axially plunged without rotation relative to housing 12, dose-setting screw 32 screws back into housing 12 as it spins relative to dose button 30. The dose markings that indicate the amount still remaining to be injected are visible through window 36. As dose-setting screw 32 screws down distally, drive member 28 is advanced distally to push piston 26 through reservoir 20 and expel medication through needle 24.

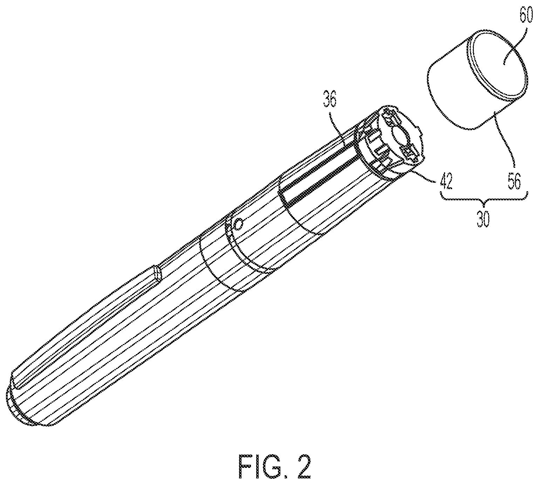

During the dose dispensing operation, the amount of medicine expelled from the medication delivery device is proportional to the amount of rotational movement of the dose-setting screw 32 relative to the housing 12 as the dose-setting screw 32 screws back into housing 12. In some embodiments, because the dose button 30 is rotationally fixed relative to the housing 12 during the dose dispensing mode, the amount of medicine expelled from the medication delivery device may be viewed as being proportional to the amount of rotational movement of the dose-setting screw 32 relative to the dose button 30 as the dose-setting 32 screws back into housing 12. The injection is completed when the internal threading of dose-setting screw 32 has reached the distal end of the corresponding outer threading of sleeve 34 (FIG. 4). Device 10 is then once again arranged in a ready state or zero dose position as shown in FIGS. 2 and 4.

As discussed above, the dose delivered may be derived based on the amount of rotation of the dose-setting assembly (flange 38 and dose-setting screw 32) relative to the actuator assembly (clutch 52 and dose button 30) during dose delivery. This rotation may be determined by detecting the incremental movements of the dose-setting assembly which are "counted" as the dose-setting assembly is rotated during dose delivery.

Further details of the design and operation of an exemplary delivery device 10 may be found in U.S. Patent No. 7,291,132, entitled Medication Dispensing Apparatus with Triple Screw Threads for Mechanical Advantage. Another example of the delivery device is an auto-injector device that may be found in U.S. Patent No. 8,734,394, entitled "Automatic Injection Device With Delay Mechanism Including Dual Functioning Biasing Member," where such device can be modified with one or more various sensor systems described herein to determine an amount of medication delivered from the medication delivery device based on the sensing of relative rotation within the medication delivery device. Another example of the delivery device is a reusable pen device that may be found in U.S. Patent No. 7,195,616, entitled "Medication Injector Apparatus with Drive Assembly that Facilitates Reset," where such device can be modified with one or more various sensor systems described herein to determine an amount of medication delivered from the medication delivery device based on the sensing of relative rotation within the medication delivery device.

Described herein is a dose detection system that may be operable to determine the amount of dose delivered based on relative rotation between a dose setting member and the device body. The dose detection system utilizes a dose setting member attached to the device body and rotatable relative to the device body about an axis of rotation during dose delivery. A sensed element is attached to and rotationally fixed with the dose setting member. An actuator is attached to the device body and is held against rotation relative to the device body during dose delivery. The sensed element thereby rotates relative to the actuator during dose delivery in relation to the amount of dose delivered.

In some embodiments, the dose detection system comprises a rotational sensor attached to the actuator assembly and a sensed element that includes surface features that are equally radially spaced about the axis of rotation of the sensed element.

In some embodiments, the dose detection systems may include a sensor and a sensed component attached to components of the medication delivery device. The term "attached" encompasses any manner of securing the position of a component to another component or to a member of the medication delivery device such that they are operable as described herein. For example, a sensor may be attached to a component of the medication delivery device by being directly positioned on, received within, integral with, or otherwise connected to, the component. Connections may include, for example, connections formed by frictional engagement, splines, a snap or press fit, sonic welding or adhesive.

The term "directly attached" is used to describe an attachment in which two components, or a component and a member, are physically secured together with no intermediate member, other than attachment components. An attachment component may comprise a fastener, adapter or other part of a fastening system, such as a compressible membrane interposed between the two components to facilitate the attachment. A "direct attachment" is distinguished from attachment where the components/members are coupled by one or more intermediate functional members.

The term "fixed" is used to denote that an indicated movement either can or cannot occur. For example, a first member is "fixed rotationally" with a second member if the two members are required to move together in rotation. In one aspect, a member may be "fixed" relative to another member functionally, rather than structurally. For example, a member may be pressed against another member such that the frictional engagement between the two members fixes them together rotationally, while the two members may not be fixed together absent the pressing of the first member.

Various sensor arrangements are contemplated herein. In general, the sensor arrangements comprise a sensor and a sensed component. The term "sensor" refers to any component which is able to detect the relative position or movement of the sensed component. The sensor may be used with associated electrical components to operate the sensor. The "sensed component" is any component for which the sensor is able to detect the position and/or movement of the sensed component relative to the sensor. For the dose detection system, the sensed component rotates relative to the sensor, which is able to detect the rotational movement of the sensed component. The sensor may comprise one or more sensing elements, and the sensed component may comprise one or more sensed elements. The sensor detects the movement of the sensed component and provides outputs representative of the movement of the sensed component.

Illustratively, the dose detection system includes an electronics assembly suitable for operation of the sensor arrangement as described herein. The medication delivery device may include a controller that is operably connected to the sensor to receive outputs from the sensor. The controller begins receiving generated signals from the sensor indicative of counts from first to last one for a total number of counts that is used for determining total displacement, e.g. angular displacement. In the case of detecting an angular movement of a dose-setting assembly, the controller may be configured to receive data indicative of the angular movement of the dose-setting assembly that can be used to determine from the outputs the amount of dose delivered by operation of the medication delivery device. The controller may be configured to determine from the outputs the amount of dose delivered by operation of the medication delivery device. The controller may include conventional components such as a processor, power supply, memory, microcontrollers, etc. Alternatively, at least some components may be provided separately, such as by means of a computer, smart phone or other device. Means are then provided to operably connect the external controller components with the sensor at appropriate times, such as by a wired or wireless connection.

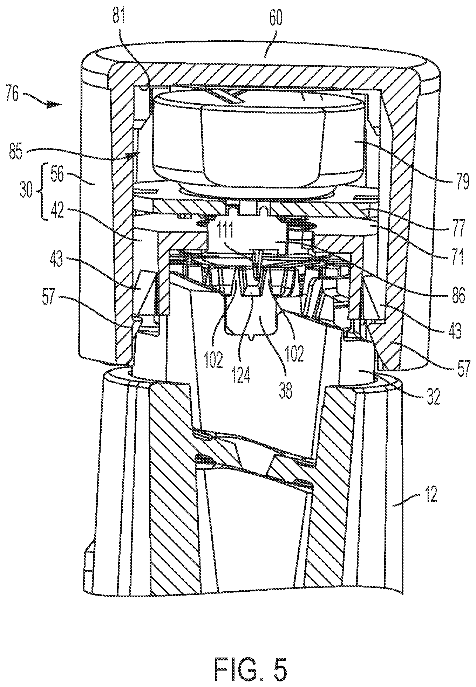

According to one aspect, the electronics assembly includes a sensor arrangement including one or more sensors operatively communicating with a processor for receiving signals from the sensor representative of the sensed rotation. An exemplary electronics assembly 76 is shown in FIGS. 5-7 and can include a sensor 86, and a printed circuit board (PCB) 77 having a plurality of electronic components. The printed circuit board may be a flexible printed circuit board. The circuit board of the electronics assembly 76 may include a microcontroller unit (MCU) as the controller comprising at least one processing core and internal memory. The electronics assembly may include a power source 79, e.g. a battery, illustratively a coin cell battery, for powering the components. The controller of electronics assembly 76 may include control logic operative to perform the operations described herein, including detecting the angular movement of the dose-setting assembly during dose setting and/or dose delivery and/or detecting a dose delivered by medication delivery device 10 based on a detected rotation of the dose-setting assembly relative to the actuator assembly. Many, if not all of the components of the electronics assembly, may be contained in a compartment 85 within the dose button 30. In some embodiments, the compartment 85 may be defined between a proximal surface 71 of support 42 of the dose button and a distal surface 81 of the cover 56 of the dose button. In the embodiment shown in FIG. 5, the electronics assembly 76 is permanently integrated within the dose button 30 of the delivery device. In other embodiments, the electronics assembly is provided as a module that can be removably attached to the actuator assembly of the medication delivery device.

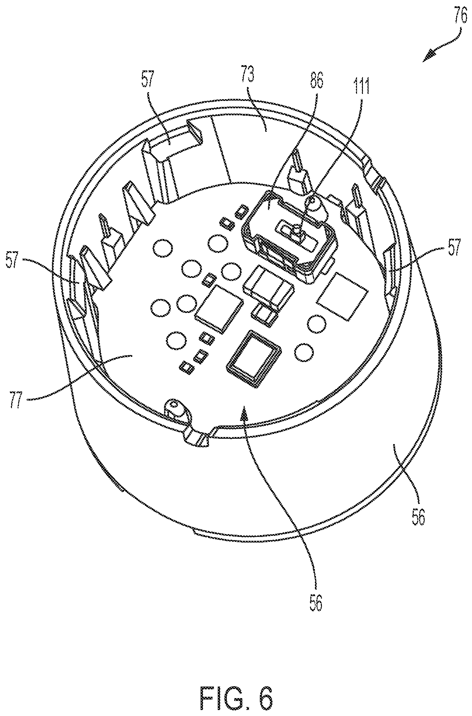

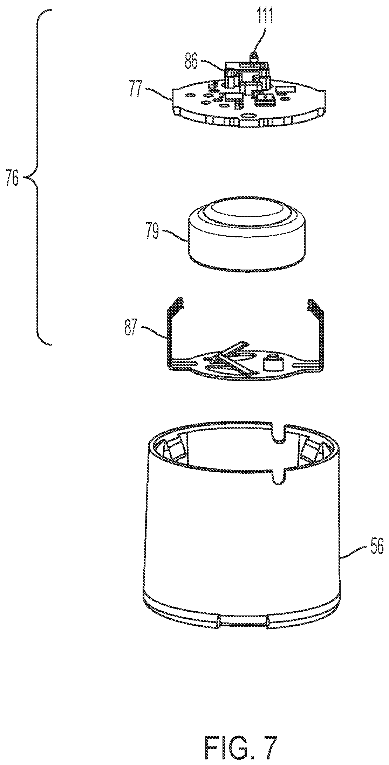

An underside view of the electronics assembly 76 held within the cover 56 is shown in FIG. 6, and an exploded view of the electronics assembly 76 is shown in FIG. 7. As shown in FIGS. 6 and 7, the electronics assembly 76 may include a printed circuit board (PCB) 77 and a sensor 86 having a contact surface 111. As shown in FIG. 7, the electronics assembly 76 may also include a battery 79 and a battery cage 87.

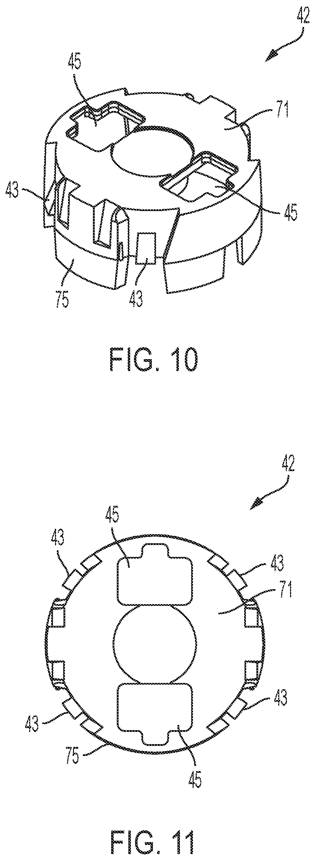

In some embodiments, at least a portion of the sensor 86 extends out of the compartment 85 of the dose button 30. As best seen in FIGS. 10 and 11, the support 42 of the dose button 30 may include one or more openings 45 through which the sensor 86 can extend through. In some embodiments, during assembly of the medication delivery device, the contact surface 111 of the sensor 86 is passed through the opening 45 of the support 42. This may permit the contact surface 111 of the sensor to interact with a component that is external to the compartment 85 of the dose button 30. In some embodiments, while only one of the openings 45 in the support 42 is needed to accommodate a sensor, a second opening may be provided, e.g. for symmetry of the support component, which help with manufacturing of the component and/or assembly of the component with the medication delivery device.

The controller of electronics assembly 76 may be operative to store the total angular movement used for determining dose delivery and/or the detected dose delivery in local memory (e.g., internal flash memory or on-board EEPROM). The controller may be further operative to wirelessly transmit a signal representative of the total counts, total angular movement, and/or detected dose to an external device, such as a user's mobile device or a remote server. Transmission may, for example, be over a Bluetooth low energy (BLE) or other suitable short or long range wireless communication protocol. Illustratively, the BLE control logic and controller are integrated on the same circuit.

As discussed, according to one aspect, the dose detection system involves detecting relative rotational movement between two assemblies of the medication delivery device. With the extent of rotation having a known relationship to the amount of a delivered dose, the sensor operates to detect the amount of angular movement from the start of a dose injection to the end of the dose injection. For example, in some embodiments, the relationship for a pen injector is that an angular displacement of a dose-setting assembly of 18° is the equivalent of one unit of dose, although other angular relationships are also suitable, such as, for example, 9, 10, 15, 20, 24 or 36 degrees may be used for a unit or a half unit. The sensor system is operable to determine the total angular displacement of a dose setting member during dose delivery. Thus, if the angular displacement is 90°, then 5 units of dose have been delivered.

The angular displacement is determined by counting increments of dose amounts as the injection proceeds. For example, a sensing system may use a repeating pattern of a sensed element, such that each repetition is an indication of a predetermined degree of angular rotation. Conveniently, the pattern may be established such that each repetition corresponds to the minimum increment of dose that can be set with the medication delivery device.

The dose detection system components may be permanently or removably attached to the medication delivery device. In some embodiments, at least some of the dose detection system components are provided in the form of a module that is removably attached to the medication delivery device. In other embodiments, the dose detection system components are permanently attached to the medication delivery device.

In some embodiments, a sensor may detect, during dose delivery, the relative rotation of a sensed component that is rotationally fixed to the dose-setting screw 32, from which is determined the amount of a dose delivered by the medication delivery device. In an illustrative embodiment, a rotational sensor is attached, and rotationally fixed, to the actuator assembly. The actuator assembly does not rotate relative to the device housing during dose delivery.

In some embodiments, a sensed component is attached, and rotationally fixed, to the dose-setting screw 32, which rotates relative to the dose button 30 and the device housing 12 during dose delivery. In some of the embodiments described herein, the sensed component includes a ring structure having a plurality of proximally extending projections circumferentially disposed relative to one another. Projections are shaped and sized to deflect a movable element of the rotational sensor. One illustrative embodiment of such a sensed component is tubular flange 38, best seen in FIGS. 3, 5, 8, and 9. Embodiments described herein may be provided for a module that is removably attachable to the dose button of the delivery device or integrated within the dose button of the delivery device.

During dose delivery, dose-setting screw 32 is free to rotate relative to dose button 30. In the illustrative embodiment, the electronics assembly 76 is rotationally fixed with the dose button 30 and does not rotate during dose delivery.

As seen in FIGS. 2, 3 and 5, the dose button 30 comprises a cover 56 coupled to a support 42. An electronics assembly 76 may be at least partially contained within a compartment 85 defined between the cover 56 and the support. In some embodiments, the cover and support have corresponding splines that engage with one another to couple the cover and support together. For example, in some embodiments, the cover 56 may couple to the support 42 via one or more snaps 57 on the cover 56 and corresponding to one or more protrusions 43 on the support. As seen in FIG. 5 and 6, the snaps 57 on the cover 56 may be directed radially inwardly from an inner circumferential sidewall 73. As seen in FIGS. 5, 10 and 11, the protrusions 43 on the support 42 may be directed radially outwardly from an outer circumferential sidewall 75 of the support 42. The protrusions 43 may form a triangular ramp shape.

The snaps 57 on the cover 56 are configured to snap over and mate with the protrusions 43 on the support to couple the cover to the support. In some embodiments, the protrusion on the support comprises a continuous annular protrusion around the outer circumferential sidewall of the support. The cover 56 may attach to the support 42 via frictional engagement, interference fit or any other suitable fit. In some embodiments, the cover 56 is permanently fixed to the support 42 during assembly, e.g. via ultrasonic welding, adhesive, or other suitable fixation approach.

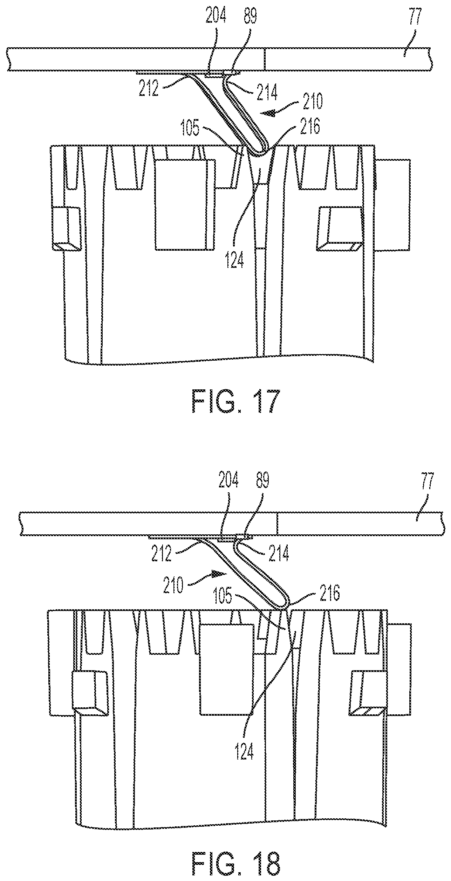

As seen in FIGS. 8 and 9, the tubular flange 38 may include a plurality of axially directed teeth 102 that are equally radially spaced about a rotation axis and arranged to correlate to the equivalent of one unit of dose. In this illustrative embodiment, the tubular flange 38 includes 20 teeth 102 that are equally rotationally spaced from one another, such that the rotation distance between two adjacent teeth corresponds to 18 degrees of rotation. Thus, with the tubular flange 38 of FIG. 8, 18 degrees of rotation of the tubular flange 38 may be used to represent one dosage unit or a half dosage unit. It should be appreciated that, in other embodiments, different total numbers of teeth may be used to create other angular relationships, such as, for example, 9, 10, 15, 18, 20, 24 or 36 degrees may be used for a unit or 0.5 unit.

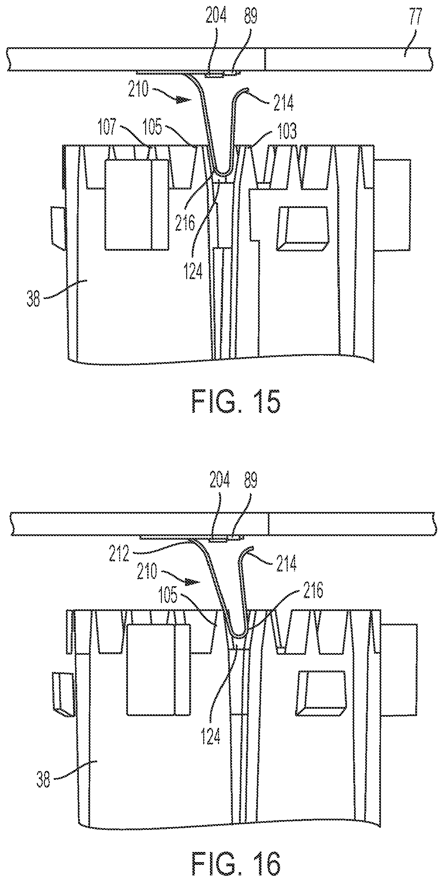

A recess 124 may be defined between each pair of adjacent teeth 102 (see FIG. 15). Each tooth 102 may have an approximately triangular shaped profile, each having a surface 120 against which a contact surface 111 of a sensor may slide.

In some embodiments, the sensor for detecting rotation of the tubular flange includes a movable element that has a contact portion capable of resting against the teeth of the tubular flange and is spring-biased such that the contact surface is configured to slide against and over the teeth during rotation of the flange relative to the actuator assembly during dose delivery. The sensor is responsive to the movement of the contact portion over the teeth and generates signals corresponding to the flange. A controller is responsive to the signals generated by the sensor to determine a dose count for determining the dosage delivered based on the detected rotation of the flange relative to the actuator assembly during dose delivery.

The contact surface may be biased against the physical features of the tubular flange to ensure proper contact between the contact surface and the physical features during rotation. In one embodiment, the movable element is a resilient member having one portion attached to the actuator at a location displaced from the contact surface. In one example, the movable element is a following member comprising a beam attached at one end to the actuator and having the contact surface at the other end. The beam is flexed to urge the contact surface in the direction of the surface features. Alternatively, the movable element may be biased in any of a variety of other ways. In addition to the use of a resilient beam, the biasing may be provided, for example, by use of a spring component. Such spring component may for example comprise a compression, tension, or torsion coil spring. In yet other embodiments, the movable element may be biased against the surface features of the sensed element by a separate resilient member or spring component bearing against the movable element.

FIG. 5 depicts an illustrative embodiment of a sensor 86 having a contact surface 111 interacting with teeth 102 of a tubular flange 38. As the flange 38 rotates relative to the dose button 30 during delivery, the teeth 102 of the flange contact and slide against the contact surface 111 of the sensor 86, causing the contact surface 111 to move in an oscillating manner. The movement of the contact surface 111 may be a combination of axial and lateral movement as the contact surface 111 slides into and out of the recesses 124 defined between the teeth 102 of the flange 38. The sensor 86 may be configured to track the movement of the contact surface 111 and associate the movement with an output signal that is sent to a controller.

As alternative to teeth on the tubular flange, surface features that interact with the sensor may comprise anything detectable by the sensor. The sensor arrangement may be based on a variety of sensed characteristics, including tactile, optical, electrical and magnetic properties, for example. In the illustrative embodiments shown in the figures, the surface features are physical features which allow for detection of incremental movements as the dose-setting assembly rotates relative to the actuator assembly. In alternative embodiments, the sensor may be a piezoelectric sensor, a magnetic sensor such as a Hall effect sensor, an accelerometer for detecting vibration, e.g. of a ratcheting or other detent mechanism, where vibration can be correlated with rotational movement, an optical sensor such as a reflective sensor, an interrupter sensor, or an optical encoder, or any other sensor suitable for sensing rotation of a first component relative to a second component.

In some embodiments, when a user presses axially on face 60 of the dose button 30, the dose button 30 advances distally relative to the housing 12, compressing spring 68. Continued pressing of the dose button 30 distally results in back driving of the dose-setting screw 32 in a spiral direction relative to housing 12. As a result, the dose-setting screw 32 and flange 38 are driven to rotate by the axially pressing upon the dose button 30. In some embodiments, the dose detection system is operable for dose detection only while the dose button is being pressed.

In some embodiments, the electronics assembly may include a clock or timer to determine the time elapsed between counts caused by trigger of the rotational sensor from the surface features of the sensed element. When no counts have been detected by the controller after a period of time this may be used to indicate that the dose has completed.

In some embodiments, a single sensing system may be employed for both dose detection sensing and wake-up activation. For example, upon the initial sensing of rotation of the sensed element by the sensor, the controller is configured to allow wake-up or activation of the electronics assembly to a greater or full power state. The wake-up feature is configured to allow power transmission from the power source (shown as battery) for powering up the electronic components for dose sensing in order to minimize inadvertent power loss or usage when a dose dispensing event is not occurring. In other embodiments, a separate wake-up switch may be provided and arranged within the dose button housing and triggered when the dose button is in its distal position. After activation of the electronics assembly, the controller begins receiving generated signals from the rotational sensor indicative of counts from first to last one for a total number of counts that is used for determining total angular displacement and thus the amount of dose delivered.

In some embodiments, the electronics assembly may have a controller that is configured to receive an output signal from a rotational sensor. The controller of the electronics assembly may be programmed to convert the intermediate signal to a conditioned digital signal, which may be a single step/square wave with a predetermined width representing a predetermined time. In some embodiments, output signals that are less than a predetermined level may be filtered out and ignored.

According to one aspect, a medication delivery device includes a repeatedly activatable switch that may serve as a sensor. In some embodiments, the switch serves as the rotational sensor in the dose detection system described above. In other embodiments, however, the switch may be used to detect other activity such as removal of a cap.

In some embodiments, the switch comprises a conductive pad and a cantilevered arm that is moveable relative to the conductive pad. The cantilevered arm may be mounted to a printed circuit board at a first end, and a second end of the arm may be unattached and free to move relative to the printed circuit board.

According to one aspect, the switch may have one or more features that help to permit the switch to repeatedly open and close. The switch may have one or more features that help the switch to avoid plastic deformation during repeated opening and closing, thus helping to maintain durability of the switch.

In some embodiments, at the first end of the cantilevered arm, where the arm attaches to the printed circuit board, the arm has a first curved portion. During switch closure, this first curved portion may be moved toward a straight configuration, and during switch opening, the straight configuration may be moved back toward the curved configuration. In the unstressed state, this first portion may be biased toward the curved configuration. Accordingly, the cantilevered arm may act as a spring that stores potential energy as it is moved during sliding interaction against a sensed component, where the stored potential energy is released to move the arm back toward the unstressed state when the sliding contact force against the arm has decreased.

In some embodiments, the cantilevered arm may transition from the first curved portion to a second curved portion that is configured to move toward and contact the conductive pad that is mounted to the PCB. Contact between the second curved portion and the conductive pad closes the switch, while lack of contact between the second curved portion and the conductive pad opens the switch.

In some embodiments, the cantilevered arm may include a third curved portion configured to contact and slide against the sensed component, e.g. against the teeth of a rotating tubular flange 38 shown in FIGS. 8 and 9. In some embodiments, the third curved portion connects the first curved portion to the second curved portion. In such embodiments, the second curved portion, which is configured to contact the conductive pad, may be located at the second end of the cantilevered arm, i.e. where the cantilevered arm truncates. In other embodiments, the second curved portion connects the first and third curved portions. In such embodiments, the third curved portion, which is configured to contact the sensed component, may be located at the second end of the cantilevered arm, i.e. where the cantilevered arm truncates.

In some embodiments, having the curved portions of the cantilevered arm may help to avoid high strain concentrations in the arm, and may thus help to prevent plastic deformation of the arm. However, it should be appreciated that one or more of the curved portions may be shaped differently in other embodiments.

In some embodiments, the switch may have one or more features that help the switch to provide a cleaner, more easily readable output signal such that a controller can more accurately identify when the switch is open and when the switch is closed. In some embodiments, a blocking protrusion may be provided to interact with the second curved portion of the cantilevered arm as the arm makes contact with the conductive pad. This blocking protrusion may be situated directly adjacent to or near the conductive pad and may prevent the arm from moving past the conductive pad. In some embodiments, the presence of the blocking protrusion may help to reduce "bounce" of the cantilevered arm that may aid in producing a cleaner output signal from the switch. In some cases, "bounce" from the cantilevered arm may cause the arm to rapidly and repeatedly contact and separate from the conductive pad in a short period of time, which can create a noisy output signal that may be difficult for a controller to interpret. The blocking protrusion may help to provide sustained contact between the cantilevered arm and the conductive pad to provide a cleaner output signal. In some embodiments, the blocking protrusion may be made from a shock-absorbing material that may help to deaden the impact of the cantilevered arm against the blocking protrusion, in order to decrease bounce or other vibration.

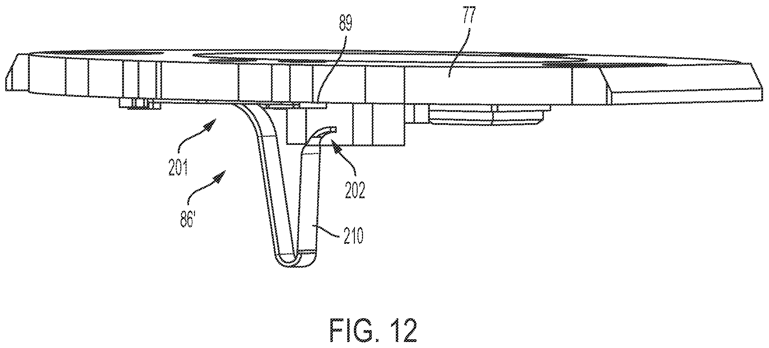

One illustrative example of a switch is shown in FIG. 12, which depicts a switch 86' having a conductive pad 89 and a cantilevered arm 210. The conductive pad 89 and a first end 201 of the cantilevered arm 210 are mounted to a PCB 77.

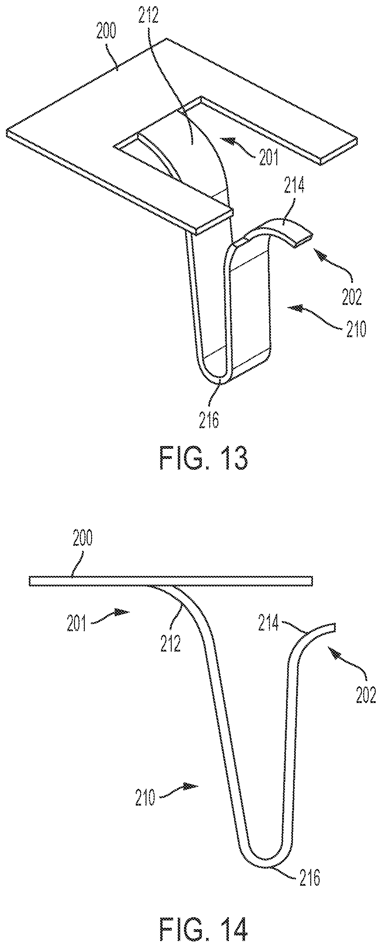

As best seen in FIGS. 13 and 14, the cantilevered arm 210 begins at a first curved portion 212 at its first end 201 and truncates at a second curved portion 214 at its second end 202. The arm also includes a U-shaped third curved portion 216 that connects the first curved portion 212 to the second curved portion 214. The second curved portion 214 is configured to contact with a conductive pad, and the third curved portion 216 is configured to contact with a sensed component such as the rotating tubular flange 38 shown in FIGS. 8 and 9.

The switch also includes a base 200 that is connected to the cantilevered arm 210. The base 200 is connected to the PCB to connect cantilevered arm to the PCB. The base and the arm together may form a single monolithic component.

FIGS. 15-19 depict the cantilevered arm 210 of the switch interacting with the rotating flange 38 from FIGS. 8 and 9. FIG. 15 shows the arm 210 in an unstressed state, as the third curved portion 216 is situated within a recess 124 between two adjacent teeth 103, 105. The switch may be positioned in this state when the flange 38 is at its home or zero dose position, e.g. prior to use of the device, prior to setting a dosage, or after a dispense has completed and the device is ready for a dosage to be set.

In FIG. 16, the flange 38 has begun to rotate relative to the switch and the PCB 77. As a result, tooth 105 slides and pushes against the third curved portion 216 of the arm 210, causing the arm 210 to begin to deflect toward a direction out of the recess 124. The first curved portion 212 begins to move toward a straightened configuration, and the second curved portion 214 begins to move toward the conductive pad 89.

In FIG. 17, the flange 38 has rotated further than in FIG. 16, causing the tooth 105 to slide against and push the third curved portion 216 nearly completely out of the recess 124. The first curved portion 212 has moved even more toward a straightened configuration. As a result, the second curved portion 214 has made contact with the conductive pad 89, thereby closing the switch. The second curved portion also pressed against a blocking protrusion 204, which prevents the second curved portion from moving further toward the first curved portion 212, and may help to prevent the second curved portion from bouncing repeatedly against the conductive pad 89 in a rapid manner that may give rise to a noisy output signal.

In FIG. 18, the flange 38 has rotated further than in FIG. 17, and the third curved portion 216 has exited the recess 124 and is sliding across the top of tooth 105. The second curved portion 214 remains in contact with both the conductive pad 89 and the blocking protrusion 204. Blocking protrusion 204 has prevented the second curved portion 214 from moving closer to the first curved portion 212.

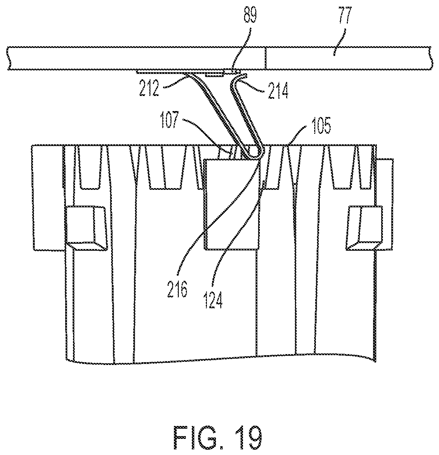

Finally, in FIG. 19, the flange 38 has rotated further than in FIG. 18, and the third curved portion 216 has stopped contacting tooth 105 and has now begun contacting the next adjacent tooth, 107. During this transition as the next tooth 107 is just beginning to push upon the arm 210, the arm, which is spring biased toward the position shown in FIG. 15, has swung back toward its unstressed state, thus causing the first curved portion 212 to move toward a more curved shape, resulting in movement of the third curved portion 216 toward a direction opposite to the rotation direction of the flange 38 and resulting in movement of the second curved portion 214 away from the conductive pad 84, thereby opening the switch. As the flange 38 rotates further, the cycle continues and the arm moves back toward the conductive pad to close the switch, and so on.

As described herein, the printed circuit board (e.g., the printed circuit board 77) can include various processing circuitry and/or logic that generates data based on the operation of the medication delivery device. For example, the processing circuitry can count the number of times the sensor (e.g., the sensor 86) is activated or triggered during an injection to determine a dose size of the injection (e.g., the dose a particular insulin injection). As described herein, the relative rotational movement between a dose-setting assembly and an actuator of the medication delivery device can be sensed in order to determine the amount of a dose delivered by a medication delivery device, because the sensed relative rotational movements can be correlated to the amount of the dose delivered.

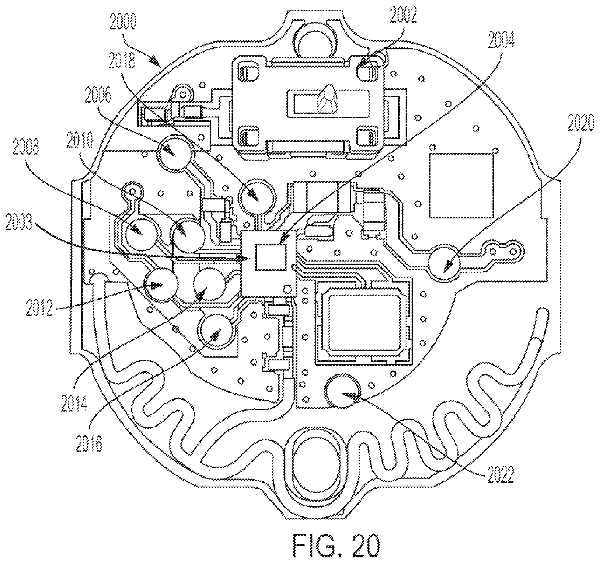

FIG. 20 is an exemplary schematic diagram of a printed circuit board 2000, according to some embodiments. The printed circuit board 2000 (e.g., printed circuit board 77) includes various components, including a sensor 2002 (e.g., sensor 86 in FIG. 6) that is in electrical communication with a microcontroller 2004 within a System-on-Chip (SoC) 2003. The printed circuit board 2000 includes a set of pads 2006, 2008, 2010, 2012, 2014, 2016, 2018, 2020 and 2022 that are in electrical communication with the microcontroller 2004 / SoC 2003. The pads can be used to connect electrical components to the microcontroller 2004 / SoC 2003, e.g., for testing, and/or the like. Some of the pads, such as pads 2008, 2010, 2012, 2014, 2016, and 2018, may not be in communication with the microcontroller 2004 / SoC 2003 by default. For example, the microcontroller / SoC may be initially programmed (e.g., via associated registers) such that some of the pads are not in electrical communication with the microcontroller 2004 (e.g., via programmable switches or resistors). One or more of the pads can be placed in electrical communication with a logic input, such as general-purpose input/output (GPIO) pin(s) of the microcontroller 2004 / SoC 2003. As an example, the microcontroller can be programmed to modify internal programmable components (e.g., one or more pull-up resistors and/or pull-down resistors) to place the pads in electrical communication with the logic input.

In some embodiments, a GPIO pin input to the microcontroller 2004 / SoC 2003 can be a logic level input. The microcontroller 2004 within SoC 2003 can detect a logical 1 if a voltage above a certain maximum threshold is applied to a GPIO pin, while the microcontroller 2004 can detect a logical 0 if a voltage below a certain minimum threshold is applied to the GPIO pin. Some pads on the printed circuit board may be connected to a voltage source. For example, pad 2020 can provide a battery voltage V_{bat}. As another example, pad 2006 can provide a voltage from a DC/DC converter V_{dcdc}.

As described herein, the microcontroller 2004 (e.g., including based on input from the sensor 2002) may be operative to process dose data and/or other data of the medication delivery device. For example, the microcontroller 2004 can be configured to store the total angular movement used for determining dose delivery and/or the detected dose delivery in local memory (e.g., internal flash memory or on-board EEPROM). The microcontroller 2004 may be further operative to wirelessly transmit a signal representative of the total counts, total angular movement, and/or detected dose to an external device, such as a user's mobile device or a remote server (e.g., via BLE control logic and controller integrated on the printed circuit board 2000).

The inventors have appreciated that if moisture is present on the printed circuit board, the moisture can affect various data of the medication delivery device. For example, moisture on the printed circuit board can cause the microcontroller to receive data indicative of dose information, when in fact no dose is occurring. As another example, moisture can cause the microcontroller to receive incorrect dose information (e.g., incorrect count information). Thus, the moisture can cause the microcontroller to sense erroneous data, which can cause the medication delivery device to process (e.g., save, transmit, etc.) the erroneous data. Accordingly, the inventors developed techniques for sensing the potential presence of moisture on a printed circuit board by detecting a voltage change. Aspects of the techniques herein leverage conductive traces (including, but not limited to, circuit pads, wires, and/or conductive materials (e.g., graphite)) of the printed circuit board 2000 and other circuitry (e.g., circuitry not normally used to sense for the presence of moisture) to determine that moisture may be present on the printed circuit board 2000. Some embodiments of the techniques described herein can connect one or more circuit pads to logical inputs to the microcontroller 2004 for use with the moisture sensing techniques described herein. For example, as described herein, one or more of the pads, such as pad 2008 and/or pad 2006, can be connected to a corresponding GPIO pin of the microcontroller 2004 (e.g., via programmable components controlled by the firmware of the microcontroller, as described herein). The pad can be biased such that the microcontroller can monitor for a change in the logic input in order to monitor for the potential presence of moisture. As another example, the techniques can use an ADC to monitor for the presence of moisture. The input of the ADC can be connected to a pad and/or other conductive trace, and the output of the ADC can be monitored by the microcontroller to determine the potential presence of moisture. The pad or trace can be biased with a voltage that allows the microcontroller to bidirectionally detect a change in the voltage output by the ADC. Upon the detection of the potential presence of moisture, the microcontroller can be configured to modify its operation accordingly (e.g., to prevent the moisture from causing the microcontroller to process erroneous data).

Aspects of the techniques described herein can be used with medication delivery devices to sense moisture. As described herein, the medication delivery device can include a housing with a reservoir that is sized sufficiently to hold a medication (e.g., insulin), and a dose button that is rotatable relative to the housing to select a dose size of the medication for an injection. The medication delivery device includes a printed circuit board with various circuitry. The circuitry can include a conductive trace. The conductive trace can be a conductive material, such as a metal (e.g., copper) and/or other conductive material (e.g., graphite). According to some examples, the conductive trace can be a conductive material (e.g., one or more of pads 2006, 2008, 2010, 2012, 2014, 2016, 2018, 2020 and 2022) that is at least partially disposed on the printed circuit board (e.g., in the plane of the printed circuit board). For example, part of the solder mask of the printed circuit board can be omitted and/or exposed to allow for fluid communication to the conductive trace(s), which can be disposed under the solder mask. According to some examples, the conductive trace can be a wire and/or other conductive material that is not disposed on the printed circuit board, such as a wire extending from the printed circuit board (e.g., through the solder mask). The circuitry can further include a bias source in electrical communication with the conductive trace, and a microcontroller in electrical communication with the conductive trace through a logic input to the microcontroller. According to some embodiments, the bias voltage can be applied to the conductive trace such that the bias can be sensed by the microcontroller at a logic interface, such as a GPIO interface.

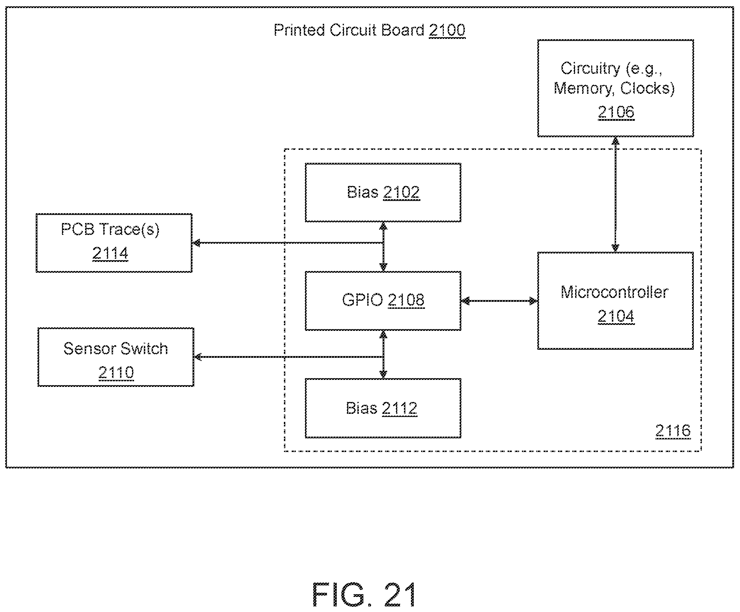

FIG. 21 shows an example of a printed circuit board 2100 with a bias source 2102, according to some embodiments. As shown in FIG. 21, the printed circuit board 2100 includes a microcontroller 2104 (e.g., a processor, such as an ARM Cortex processor) that is in electrical communication with various circuitry 2106 (e.g., memory, clock(s), etc.), and a set of GPIOs 2108. The sensor switch 2110 is in electrical communication with a first bias source 2112 and a logical input of the microcontroller, which in this example is one of the GPIOs 2108. In accordance with some embodiments of the techniques described herein, PCB trace(s) 2114 can be in electrical communication with the bias source 2102 and the GPIO 2108. According to some embodiments, the bias source 2102 can be a resistor. The resistor can be a pull-up resistor (e.g., connected to V_{bat}, or to a voltage source corresponding to a logical 1) or a pulldown resistor (e.g., connected to ground). The printed circuit board 2100 can, in some embodiments, include a System on Chip (SoC) (e.g., SoC 2003), as illustrated by the dotted box 2116. While the microcontroller 2104, GPIO 2108, bias source 2102 and bias source 2112 are shown as part of the SoC 2116, this is for illustrative purposes only. For example, one or more of the microcontroller 2104, GPIOs 2108, bias source 2102, and/or bias source 2112 can be additional components on the printed circuit board 2100 that are not included as part of the SoC 2116. In embodiments that do not use a SoC 2116, one or more of microcontroller 2104, GPIOs 2108, and bias source 2102 may be mounted on printed circuit board 2100 as discrete components.

The bias source, such as a resistor, can be internal or external to the SoC. According to some embodiments, one or more resistors are used as the bias source 2102 that are external to the SoC 2116. According to some embodiments, one or more resistors are used as the bias source 2102 that are internal to and/or part of the SoC 2116. For example, one or more on-silicon (e.g., system on chip (SoC) integrated circuit) programmable bias resistors can be used to create the bias source 2102. Whether to use internal resistor(s) or external resistor(s) can be a matter of design consideration. For example, if it is desirable to select the bias resistor(s), then using external resistors can allow for the selection of the resistor properties of the resistor(s) that are added to the printed circuit board 2000. For example, assume that the built-in resistors available in the SoC 2116 have a resistance of 25 kohms. If it is desirable to use resistors with properties that allow for the detection of liquids at conductivities that may not be detected if using such a resistor, then external resistor(s) can be used and added to the printed circuit board 2110 accordingly. Selection of appropriate bias resistors may therefore allow for incorporation of an early detection capability better than that available if using programmable bias resistors within the SoC 2116. However, adding additional bias resistor(s) may increase the space used on the printed circuit board 2000 and/or may increase the cost of materials and/or manufacturing. In contrast, using programmable bias resistors internal to the SoC 2116 may not require using any additional space on the printed circuit board 2000, and may not increase the cost of materials or manufacturing. Further, continuing with the example of available internal resistor(s) at 25 kohms, such resistors may be sufficient for detecting moisture since the amount of water / type of water that would cause the switch 2002 to trip would also cause GPIOs that are connected to pads with a bias voltage applied by internal resistors to also trip.

In some embodiments, bias source 2102 and/or bias source 2112 may be any electrical component with a small but measurable leakage current, and that can serve as a weak current source. Such an electrical component may be a resistor, as previously described, but may also be a diode, a transistor, and/or a capacitor. In general, bias source 2102 and/or bias source 2112 may be any electrical component that (i) conducts a small but measurable leakage current sufficient to pull GPIO 2108 to a specified voltage in the absence of moisture but (ii) does not conduct so much current so as to prevent GPIO 2108 from being biased to another, detectable voltage level in the presence of moisture. In some cases, it may be easier and/or more efficient to use electrical components already embedded and/or integrated in a SoC, e.g., diodes, transistors, and/or capacitors, rather than adding additional resistors.

According to some configurations of the printed circuit board 2000, one or more of the circuit traces and/or pads may not be connected to the GPIOs by default (e.g., and are thus available for use, if desired). The techniques can include connecting such GPIOs to the microcontroller (e.g., via programming of the microcontroller 2004). According to some embodiments, the default configuration of one or more unused GPIO pins may be as an "output" instead of an "input." Therefore, the techniques may also include modifying the microcode of the microcontroller 2004 to change the GPIO pin(s) used according to the techniques described herein to "inputs" to the microcontroller 2004. Simply connecting a pad to a GPIO, however, may allow the voltage from that pad to float at any value. According to some embodiments, as described herein the bias is provided to the pad connected to the GPIO to allow for detection of voltage sources.

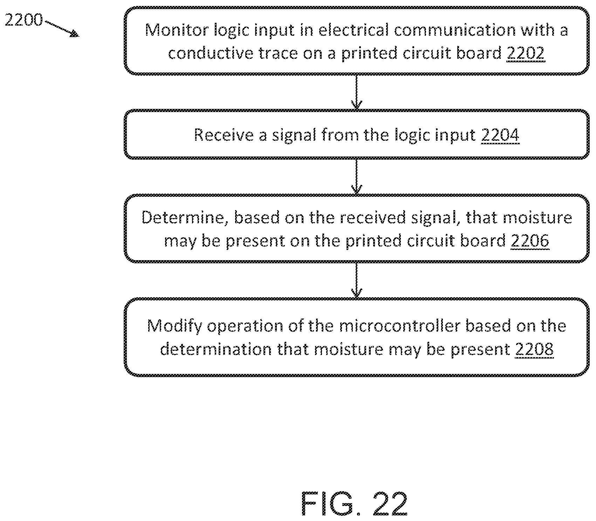

FIG. 22 is a flow chart showing a first exemplary computerized method 2200 that can be executed by the microcontroller of a medication delivery device to determine whether there is likely moisture present in the medication delivery device, according to some embodiments. At step 2202, the microcontroller monitors a logic input in electrical communication with a conductive trace on the printed circuit board. At step 2204, the microcontroller receives a signal from the logic input. At step 2206, the microcontroller determines, based on the received signal, that moisture may be present on the printed circuit board. At step 2208, the microcontroller can modify its operation based on the determination that moisture may be present on the printed circuit board.

Referring to step 2202, as described herein a pad on the printed circuit board can be appropriately voltage biased so that in normal, non-fault operating mode, the trace is held in a state that allows the microcontroller firmware to read an expected logical input via the GPIO. The bias voltage can be configured according to various voltages. For example, the bias voltage can be to ground and/or to an available voltage that can be read by the GPIO logic level (e.g., in accordance with specifications of the electronics used for the techniques described herein). For example, if the bias is a positive voltage (e.g., V_{bat}), the microcontroller can expect to read a logical 1 under normal operating conditions. The positive voltage can be, for example, 1 volt, 1.5 volts, 2 volts, and/or the like. As another example, if the bias is to ground (e.g., approximately 0 volts), the microcontroller can expect to read a logical 0 during normal operating conditions.

Referring to steps 2204-2206, if the microcontroller reads a voltage from the GPIO at the expected logical level, normal firmware operation is allowed to progress. However, if the microcontroller detects that the GPIO is being pulled away from the normal biased voltage state by more than a predetermined voltage threshold, the microcontroller can determine a fault condition may exist on the printed circuit board. For example, the fault condition can be caused by a conductive contaminant (e.g., conductive liquid, condensing humidity, etc.) in contact with the printed circuit board traces and electrically connecting the traces to other components (e.g., to ground and/or a voltage source) that are normally not electrically connected to the traces when the conductive contaminant is not present. If the voltage applied to the GPIO changes from the expected voltage level by greater than a threshold amount, the microcontroller can read the change in voltage on GPIO as a change in state of the logic level of the GPIO, and the firmware can enter a fault state that can modify normal firmware operation (e.g., to adjust to the fact that data may be erroneous, and therefore to prevent saving and/or reporting contemporaneously delivered drug information). For example, if a GPIO is biased to ground (e.g., such that the microcontroller 2004 normally reads a logical 0), if a liquid conducts the pad to a layout local voltage of a sufficiently high voltage such that the voltage on the GPIO increases by more than a threshold amount, the microcontroller will read a logical 1. Such a change from an expected logical 0 to a logical 1 can cause the microcontroller to determine that there is the potential presence of moisture. As another example, if a GPIO is biased to a positive voltage (e.g., such that the microcontroller 2004 expects to read a logical 1), a ground conductive liquid can decrease the voltage on the GPIO by more than a threshold amount so as to cause the processor to read a logical 0 and cause a detect condition. Such a change from an expected logical 1 to a logical 0 can cause the microcontroller to detect the potential presence of moisture.

At step 2208, according to some embodiments, the microcontroller can be configured to take one or more actions upon determining that there may be moisture on the printed circuit board. For example, an algorithm programmed in the microcontroller can be configured to trigger a failsafe condition. According to some embodiments, the microcontroller can be configured to not send some information that the microcontroller would normally send to an external device. For example, the microcontroller can be configured to not send (e.g., via Bluetooth) information related to an injection, such as count information, dose information, the occurrence of an injection, and/or the like. For example, as described herein, the medication delivery device can include a switch mounted to the printed circuit board, wherein the microcontroller is configured to receive a set of signals from the switch. According to some embodiments, the switch is actuated by the rotation of a rotatable element that has a series of protrusions that are spaced from one another, and the rotatable element is positioned to permit the protrusions to slide against the switch to move the switch between a closed position and an open position as the rotatable element rotates. The microcontroller generates, based on the set of received signals, a count of the set of signals. According to some embodiments, the microcontroller can be configured to not save some information that the microcontroller would save during normal operation. For example, the microcontroller can discard a generated count of signals from the switch, such that the generated count is not stored in a memory in communication with the microcontroller.

In some embodiments, the medication delivery device can include one or more aspects that control whether the medication delivery device stores and/or discards data that would be written to non-volatile memory. For example, the medication delivery device can be configured to include a field or flag that the controller checks prior to writing data, such that the flag can be used to control whether the controller writes data to the non-volatile memory or discards the data without writing it to the non-volatile memory.

According to some embodiments, the microcontroller can be configured to shut down, restart, and/or to enter into a lower power state (e.g., a hibernation state). According to some embodiments, the microcontroller can be configured to transmit data to one or more external devices. For example, the microcontroller can be configured to transmit Bluetooth data indicative of the medication delivery device potentially having moisture on the printed circuit board. Such transmitted data can cause the receiving device to perform one or more actions, such as ignoring recently received data, providing error warnings, and/or the like.

According to some embodiments, the microcontroller can, additionally or alternatively, be configured to monitor for a voltage, generally, and to perform a particular action (e.g., a reset) upon the detection of a voltage. For example, the microcontroller can monitor one or more of the pads for a voltage (e.g., V_{bat}, which can occur if water electrically connects a pad to V_{bat}). If the microcontroller detects a sufficient voltage (e.g., a voltage above a threshold, such as ½ V_{bat}), it can cause the microcontroller to reset, power down, modify the firmware operation, and/or the like.

According to some embodiments, a plurality of GPIOs can be connected to different traces with the same and/or different bias voltages. For example, multiple pads can be connected to associated GPIO pins and be biased with positive voltages. Using multiple traces in such a manner may increase the probability of detecting moisture (e.g., since using multiple pads can increase the area of the printed circuit board 2000 across which moisture can be detected).

According to some embodiments, other conductive traces surrounding the conductive trace to which the GPIO is connected may be biased to a voltage different from the voltage to which the GPIO-connected trace is biased. For instance, if the GPIO is connected to pad 2014 and pad 2014 is biased to a positive voltage (e.g., V_{bat}), then other pads, such as pads adjacent or close to pad 2014 (e.g., pad 2008, 2010, 2012, and/or 2016) may be connected to ground. Conversely, if pad 2014 is biased to approximately 0 volts such that the microcontroller connected to pad 2014 reads a logical 0 in the absence of moisture, then other pads, such as pads adjacent or close to pad 2014 (e.g., pad 2008, 2010, 2012, and/or 2016) may be connected to a positive voltage (e.g., V_{bat}). Biasing pads that surround the connected conductive trace to an opposite voltage increases the likelihood that any moisture on the PCB will be detected more quickly and/or more reliably, because there are more opportunities for moisture to connect the GPIO-connected trace (e.g., pad 2014) to another voltage source that pulls the GPIO-connected trace away from its default voltage.

According to some embodiments, the circuitry used to monitor for moisture can include an analog-to-digital converter (ADC). The microcontroller can use the ADC to detect potential moisture on the printed circuit board. For example, the microcontroller can monitor an analog-to-digital steady state voltage of the ADC to detect a change in the voltage, which can be indicative of a potential moisture condition. According to some embodiments, an ADC (e.g., with a high impedance input node, as described herein) can be included on the printed circuit board 2000 shown in FIG. 20. The input of the ADC can be connected to one or more traces on the printed circuit board 2000, and the output of the ADC can be connected to the microcontroller 2004. According to some embodiments, a bias voltage can be applied at the input interface to the ADC. The ADC can be configured to output a word based on the input voltage to the ADC. The word can be, for example, 9 bits, 10 bits, 11 bits, and/or the like. The microcontroller 2004 can monitor the output of the ADC for a voltage change, which could be indicative of the presence of moisture as described herein. For example, if the output of the ADC can range from 0 to 1023 decimal, an input voltage of 0.45 volts, which is ½ of the operating range of the ADC, can result in the ADC outputting a value at or near 3FF hex. If the word received from the ADC is at or near an expected value (e.g., 3FF hex), the microcontroller 2004 can continue under normal firmware operation. If, instead, the ADC is pulled away from the normal biased voltage state, the output value can be significantly higher and/or lower than the expected value (e.g., close to 1023 and/or 0), which can be indicative of a fault condition being applied to the PCB traces by a conductive material (e.g., conductive liquid, condensing humidity, etc. as described herein). The microcontroller can read the ADC output, observe the change in output value, and change from normal firmware operation (e.g., to prevent reporting contemporaneously delivered drug information to the end user).

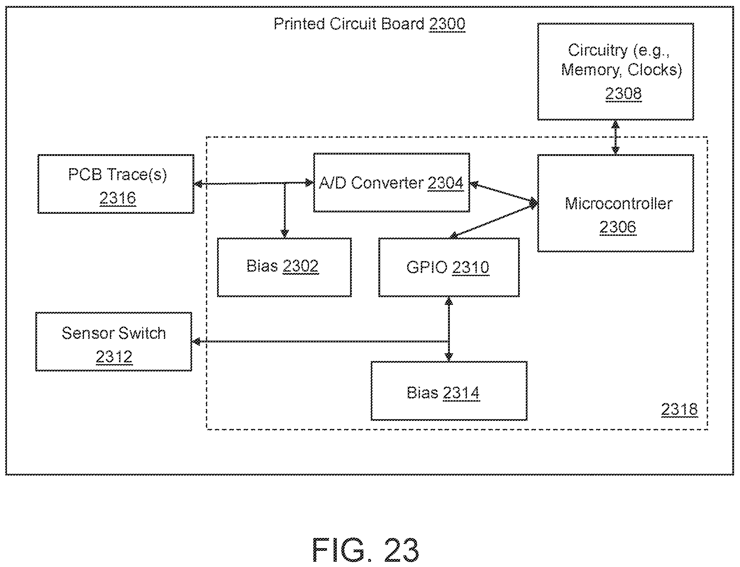

FIG. 23 shows an example of a printed circuit board 2300 with a bias source 2302 and an ADC 2304, according to some embodiments. As shown in FIG. 23, the printed circuit board 2300 includes a microcontroller 2306 that is in electrical communication with various circuitry 2308 (e.g., memory, clock(s), etc.), and a set of GPIOs 2310. The sensor switch 2312 is in electrical communication with a first bias source 2314 and the GPIOs 2310. In accordance with some embodiments of the techniques described herein, the input of the ADC 2304 can be in electrical communication with the PCB trace(s) 2316 and the bias source 2302, and the output is in electrical communication with the microcontroller 2306. As described herein, according to some embodiments, the bias source 2302 can be any electrical component described previously in relation to bias sources 2102 and/or 2112, e.g., a resistor, diode, transistor, capacitor, and/or other impedance source.

The printed circuit board 2300 can, in some embodiments, includes a SoC (e.g., SoC 2003), as shown by the dotted box 2318. While the ADC 2304, microcontroller 2306, GPIO 2310, bias source 2302, and bias source 2314 are shown as part of the SoC 2318 in FIG. 23, this is for illustrative purposes only. For example, according to some embodiments, one or more resistors and/or ADCs are used that are external to the SoC 2318. According to some embodiments, one or more ADCs and/or resistors can be used that are internal to and/or part of the SoC 2318. In embodiments that do not use a SoC 2318, one or more of microcontroller 2306, ADC 2304, GPIO 2310, bias source 2302, and/or bias source 2314 may be mounted on printed circuit board 2300 as discrete components. As described herein, whether to use internal or external bias sources, such as internal or external resistors, can be a matter of design choice (e.g., including resistor selection, use of PCB space, cost, etc.). According to some embodiments, for example, using external resistors with an ADC may allow for a unidirectional or bidirectional detection method (e.g., with an external resistor network that biases the ADC input voltage to ½ voltage ADC input measurement range). As another example, using internal programmable bias resistors may only allow for unidirectional detection (e.g., depending upon the programmability and/or capability of the internal resistors). According to some embodiments, using an internal resistor may allow for an early detection capability based upon a finer voltage measurement resolution (e.g., versus GPIO method only, since a GPIO only allows for detection of two logical states).

According to some embodiments, a separate bias source is not needed with the ADC. For example, an internal capacitive bias can be used to bias the ADC input voltage to ½ of the voltage of the ADC measurement range. According to some embodiments, a SoC integrated circuit that contains an ADC input stage that is high impedance and capacitively coupled (e.g., such that only a small PCB trace for water detection is routed from the ADC input to the PCB) can allow for the ADC to be at a voltage of approximately ½ of the voltage of a normal steady state reading.

The ADC 2304 can be in electrical communication with the microcontroller in various configurations. According to some embodiments, the ADC 2304 can share a path to the microcontroller with one or more other components (e.g., the circuitry can include a multiplexer that multiplexes the ADC 2304 output with a pin used by the GPIO 2310 block, such that the processor can switch between the ADC 2304 and the GPIO 2310 block). According to some embodiments, the ADC 2304 has its own path to the microcontroller (e.g., that is not shared with other circuitry).

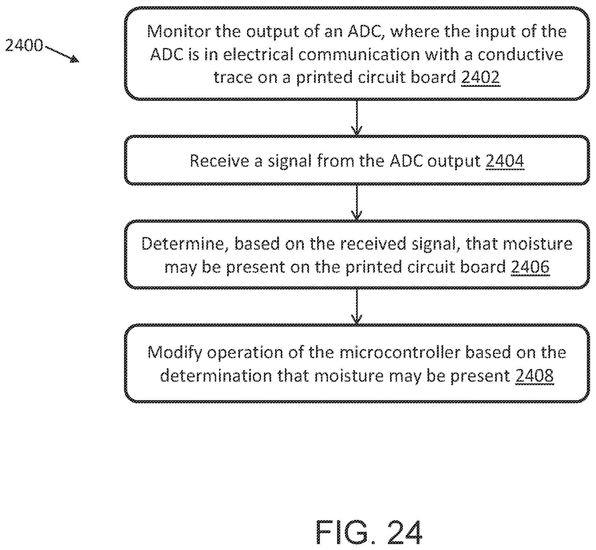

FIG. 24 is a flow chart showing a first exemplary computerized method 2400 that can be executed by the microcontroller of a medication delivery device to determine whether there is likely moisture present in the medication delivery device, according to some embodiments. At step 2402, the microcontroller monitors the output of the ADC, where the input of the ADC is in electrical communication with a conductive trace that is at least partially disposed on the printed circuit board. At step 2404, the microcontroller receives a signal from the ADC output. At step 2406, the microcontroller determines, based on the received signal, that moisture may be present on the printed circuit board. At step 2408, the microcontroller can modify its operation based on the determination that moisture may be present on the printed circuit board.

Referring to step 2402, the microcontroller can monitor the output of an ADC for an expected voltage. The ADC can be configured to operate at an operating input range that is between a low input voltage (e.g., a lower rail) and a high input voltage (e.g., an upper rail). Thus, according to some embodiments, that ADC can operate according to two rails that define a range of voltages that can be received by the ADC to feed in to the analog stage (and ultimately be converted to digital values). According to some embodiments, the low input voltage can be a ground voltage (e.g., approximately 0 volts, where the voltage is a voltage that the controller recognizes as ground), and the high input voltage can be a positive voltage (e.g., a power supply voltage, such as V_{bat}). For example, V_{bat} can be 1.0 volts, 1.5 volts, 2 volts, etc. According to some embodiments, a regulator can be used to reduce V_{bat} down to a desired upper voltage for the ADC, such as to reduce 1.5 volts down to 0.9 volts. As described herein, the ADC outputs a word value based on the input voltage.

Referring to step 2404, according to some embodiments the ADC input can be biased using a resistance or impedance source. The bias voltage provided by the bias source can be a percentage of the operating input range of the ADC, such as between 25% and 75%, or between 40% and 60%, of the operating input range of the ADC. According to some embodiments, the bias voltage is approximately 50% of the operating voltage range of the ADC. For example, if the ADC input can range from 0.0 to 0.9 volts, the ADC input can be biased to ½ of 0.9 volts, or 0.45 volts. According to some embodiments, the resistance or impedance can be configured such that it is high compared to the expected resistance or impedance of a liquid conductive path on the printed circuit board to either ground or the voltage supply (e.g., V_{bat}) if moisture is present on the printed circuit board. According to some embodiments, the percentage of the ADC operating range (e.g., ½ V rail) can be created using two megaohm scale resistors, which can be of equal value and/or ratio scaled values, as appropriate. For example, one resistor can pull to the higher voltage Vᵣₐᵢₗ (e.g., 0.9 volts), and the second resistor can pull to ground, such as to create a voltage divider (in this example, dividing to one half of the ADC input range). Since the resistance of the biasing resistors are high compared to the expected resistance or impedance of a liquid conductive path to either ground or the voltage supply (e.g., V_{bat}) if moisture is present, the biasing resistors should not prevent the input to the ADC from being pulled to either ground or the voltage supply if moisture is present. According to some embodiments, the percentage of the operating range can be created using a capacitive coupling that uses leakage current to drive to a percentage of the voltage rail.

Referring to step 2406, the microcontroller determines, based on the received signal, that moisture may be present on the printed circuit board. Adding a bias to the ADC input can allow the microcontroller to detect the presence of moisture in a bidirectional manner, such that the impedance input at the configured percentage of the voltage rail can be upset in the event of conductive liquid ingress. For example, assume the bias is configured to bias the ADC input voltage to ½ of the voltage of the ADC input measurement range. Under normal conditions, the microcontroller can expect to receive a value from the ADC indicative of the normal operating voltage input to the ADC being at ½ voltage normal steady state. If the ADC is also biased with a fault (e.g., via moisture) to either ground or to one or more other voltage source(s), the microcontroller can detect a movement of the ADC output away from the ½ voltage normal steady state (e.g., towards the lower or upper output values of the ADC), which can allow the microcontroller to detect the possible presence of a conductive fluid. Therefore, if water on the printed circuit board only connects the PCB trace to ground, the microcontroller can detect a drop in the input ADC voltage. Similarly, if the water only connects the PCB trace to V_{bat}, then the microcontroller can similarly detect that increase voltage change input to the ADC. Thus, when liquid of some conductivity upsets the balance of the bias, the ADC can measure that balance being shifted and the microcontroller can handle that detection accordingly.

According to some embodiments, one or more thresholds can be used to determine whether or not a change of the ADC output is indicative of a fault condition. For example, a change from the expected output that is greater than 3/8 of the possible ADC output range from the expected output at the steady state can be indicative of a fault condition. For example, if the steady state typically outputs a value at approximately ½ of the output range, an output value that is below 1/8 of the output range or above 7/8^{th} of the output range can be indicative of a fault. As another example, a threshold of ¼ of the possible output range from the expected output can be used, such that for a steady state that outputs a value at approximately ½ of the output range, an output value that is below ¼ of the output range or above ¾ of the output range can be indicative of a fault.

Referring to step 2408, the microcontroller can modify its operation based on the determination that moisture may be present on the printed circuit board. For example, one or more of the modifications discussed in conjunction with step 2208 of FIG. 22 can be used, according to some embodiments.

According to some examples, when using a logical input such as a GPIO as described herein, there may be a range of voltages between a minimum voltage threshold (below which the microcontroller will detect a logical 0) and a maximum threshold (above which the microcontroller will detect a logical 1) that may be indeterminate. Since the ADC outputs a word (e.g., a 9, 10 or 11 bit word), the ADC can provide for more granular monitoring. However, using an ADC may require adding the ADC to the printed circuit board. Using an ADC may additionally or alternatively require more complex programming of the microcontroller compared to using a logical input. Therefore, using a logic input may use less space, may require less complex programming, and/or the like.

According to some embodiments, the moisture detection techniques described herein (e.g., using a GPIO and/or ADC) can include one or more additional features. According to some embodiments, one or more additional nodes (e.g., other than the PCB trace(s) used for detection) can be exposed to allow for further conductive liquid paths to the PCB trace(s) used for detection. The nodes can be exposed, in some embodiments, without including a solder mask. According to some embodiments, test points (e.g., a circuit pad) adjacent to the PCB trace(s) used for detection can be biased to a voltage different from that to which the PCB trace(s) used for detection is biased. Such test points can be biased with internal and/or external resistors. Biasing such test points can improve conditions for detecting a fault condition (e.g., by providing for a known steady-state for normal operation, which can allow for easier detection of state changes). Such techniques can, for example, enhance the ability of the detection circuitry to detect moisture, as there are more voltage sources to which the moisture may connect the PCB trace(s) used for detection and thus trigger a fault condition.

The shown device is a reusable pen-shaped medication injection device, generally designated, which is manually handled by a user to selectively set a dose and then to inject that set dose. Injection devices of this type are well known, and the description of device is merely illustrative as the sensing system can be adapted for use in variously configured medication delivery devices, including differently constructed pen-shaped medication injection devices, differently shaped injection devices, and infusion pump devices. The medication may be any of a type that may be delivered by such a medication delivery device. The shown device is intended to be illustrative and not limiting as the sensing system described further below may be used in other differently configured devices.

Techniques operating according to the principles described herein may be implemented in any suitable manner. The processing and decision blocks of the flow charts above represent steps and acts that may be included in algorithms that carry out these various processes. Algorithms derived from these processes may be implemented as software integrated with and directing the operation of one or more single- or multi-purpose processors, may be implemented as functionally-equivalent circuits such as a Digital Signal Processing (DSP) circuit or an Application-Specific Integrated Circuit (ASIC), or may be implemented in any other suitable manner. It should be appreciated that the flow charts included herein do not depict the syntax or operation of any particular circuit or of any particular programming language or type of programming language. Rather, the flow charts illustrate the functional information one skilled in the art may use to fabricate circuits or to implement computer software algorithms to perform the processing of a particular apparatus carrying out the types of techniques described herein. It should also be appreciated that, unless otherwise indicated herein, the particular sequence of steps and/or acts described in each flow chart is merely illustrative of the algorithms that may be implemented and can be varied in implementations and embodiments of the principles described herein.

Accordingly, in some embodiments, the techniques described herein may be embodied in computer-executable instructions implemented as software, including as application software, system software, firmware, middleware, embedded code, or any other suitable type of computer code. Such computer-executable instructions may be written using any of a number of suitable programming languages and/or programming or scripting tools, and also may be compiled as executable machine language code or intermediate code that is executed on a framework or virtual machine.

When techniques described herein are embodied as computer-executable instructions, these computer-executable instructions may be implemented in any suitable manner, including as a number of functional facilities, each providing one or more operations to complete execution of algorithms operating according to these techniques. A "functional facility," however instantiated, is a structural component of a computer system that, when integrated with and executed by one or more computers, causes the one or more computers to perform a specific operational role. A functional facility may be a portion of or an entire software element. For example, a functional facility may be implemented as a function of a process, or as a discrete process, or as any other suitable unit of processing. If techniques described herein are implemented as multiple functional facilities, each functional facility may be implemented in its own way; all need not be implemented the same way. Additionally, these functional facilities may be executed in parallel and/or serially, as appropriate, and may pass information between one another using a shared memory on the computer(s) on which they are executing, using a message passing protocol, or in any other suitable way.

Generally, functional facilities include routines, programs, objects, components, data structures, etc. that perform particular tasks or implement particular abstract data types. Typically, the functionality of the functional facilities may be combined or distributed as desired in the systems in which they operate. In some implementations, one or more functional facilities carrying out techniques herein may together form a complete software package. These functional facilities may, in alternative embodiments, be adapted to interact with other, unrelated functional facilities and/or processes, to implement a software program application.

Some exemplary functional facilities have been described herein for carrying out one or more tasks. It should be appreciated, though, that the functional facilities and division of tasks described is merely illustrative of the type of functional facilities that may implement the exemplary techniques described herein, and that embodiments are not limited to being implemented in any specific number, division, or type of functional facilities. In some implementations, all functionality may be implemented in a single functional facility. It should also be appreciated that, in some implementations, some of the functional facilities described herein may be implemented together with or separately from others (i.e., as a single unit or separate units), or some of these functional facilities may not be implemented.

Computer-executable instructions implementing the techniques described herein (when implemented as one or more functional facilities or in any other manner) may, in some embodiments, be encoded on one or more computer-readable media to provide functionality to the media. Computer-readable media include magnetic media such as a hard disk drive, optical media such as a Compact Disk (CD) or a Digital Versatile Disk (DVD), a persistent or non-persistent solid-state memory (e.g., Flash memory, Magnetic RAM, etc.), or any other suitable storage media. Such a computer-readable medium may be implemented in any suitable manner. As used herein, "computer-readable media" (also called "computer-readable storage media") refers to tangible storage media. Tangible storage media are non-transitory and have at least one physical, structural component. In a "computer-readable medium," as used herein, at least one physical, structural component has at least one physical property that may be altered in some way during a process of creating the medium with embedded information, a process of recording information thereon, or any other process of encoding the medium with information. For example, a magnetization state of a portion of a physical structure of a computer-readable medium may be altered during a recording process.

Further, some techniques described above comprise acts of storing information (e.g., data and/or instructions) in certain ways for use by these techniques. In some implementations of these techniques-such as implementations where the techniques are implemented as computer-executable instructions-the information may be encoded on a computer-readable storage media. Where specific structures are described herein as advantageous formats in which to store this information, these structures may be used to impart a physical organization of the information when encoded on the storage medium. These advantageous structures may then provide functionality to the storage medium by affecting operations of one or more processors interacting with the information; for example, by increasing the efficiency of computer operations performed by the processor(s).

In some, but not all, implementations in which the techniques may be embodied as computer-executable instructions, these instructions may be executed on one or more suitable computing device(s) operating in any suitable computer system, or one or more computing devices (or one or more processors of one or more computing devices) may be programmed to execute the computer-executable instructions. A computing device or processor may be programmed to execute instructions when the instructions are stored in a manner accessible to the computing device or processor, such as in a data store (e.g., an on-chip cache or instruction register, a computer-readable storage medium accessible via a bus, a computer-readable storage medium accessible via one or more networks and accessible by the device/processor, etc.). Functional facilities comprising these computer-executable instructions may be integrated with and direct the operation of a single multi-purpose programmable digital computing device, a coordinated system of two or more multi-purpose computing device sharing processing power and jointly carrying out the techniques described herein, a single computing device or coordinated system of computing device (co-located or geographically distributed) dedicated to executing the techniques described herein, one or more Field-Programmable Gate Arrays (FPGAs) for carrying out the techniques described herein, or any other suitable system.

A computing device may comprise at least one processor, a network adapter, and computer-readable storage media. A computing device may be, for example, a desktop or laptop personal computer, a personal digital assistant (PDA), a smart mobile phone, a server, or any other suitable computing device. A network adapter may be any suitable hardware and/or software to enable the computing device to communicate wired and/or wirelessly with any other suitable computing device over any suitable computing network. The computing network may include wireless access points, switches, routers, gateways, and/or other networking equipment as well as any suitable wired and/or wireless communication medium or media for exchanging data between two or more computers, including the Internet. Computer-readable media may be adapted to store data to be processed and/or instructions to be executed by processor. The processor enables processing of data and execution of instructions. The data and instructions may be stored on the computer-readable storage media.

A computing device may additionally have one or more components and peripherals, including input and output devices. These devices can be used, among other things, to present a user interface. Examples of output devices that can be used to provide a user interface include printers or display screens for visual presentation of output and speakers or other sound generating devices for audible presentation of output. Examples of input devices that can be used for a user interface include keyboards, and pointing devices, such as mice, touch pads, and digitizing tablets. As another example, a computing device may receive input information through speech recognition or in other audible format.

Embodiments have been described where the techniques are implemented in circuitry and/or computer-executable instructions. It should be appreciated that some embodiments may be in the form of a method, of which at least one example has been provided. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

Various aspects of the embodiments described above may be used alone, in combination, or in a variety of arrangements not specifically discussed in the embodiments described in the foregoing and is therefore not limited in its application to the details and arrangement of components set forth in the foregoing description or illustrated in the drawings. For example, aspects described in one embodiment may be combined in any manner with aspects described in other embodiments.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

Also, the phraseology and terminology used herein is for the purpose of description and should not be regarded as limiting. The use of "including," "comprising," "having," "containing," "involving," and variations thereof herein, is meant to encompass the items listed thereafter and equivalents thereof as well as additional items.

The word "exemplary" is used herein to mean serving as an example, instance, or illustration. Any embodiment, implementation, process, feature, etc. described herein as exemplary should therefore be understood to be an illustrative example and should not be understood to be a preferred or advantageous example unless otherwise indicated.

To clarify the use of and to hereby provide notice to the public, the phrases "at least one of <A>, <B>, . . . and <N>" or "at least one of <A>, <B>, . . . <N>, or combinations thereof" or "<A>, <B>, . . . and/or <N>" are defined by the Applicant in the broadest sense, superseding any other implied definitions hereinbefore or hereinafter unless expressly asserted by the Applicant to the contrary, to mean one or more elements selected from the group comprising A, B, . . . and N. In other words, the phrases mean any combination of one or more of the elements A, B, . . . or N including any one element alone or the one element in combination with one or more of the other elements which may also include, in combination, additional elements not listed.

While various embodiments have been described, it will be apparent to those of ordinary skill in the art that many more embodiments and implementations are possible. Accordingly, the embodiments described herein are examples, not the only possible embodiments and implementations. Furthermore, the advantages described above are not necessarily the only advantages, and it is not necessarily expected that all of the described advantages will be achieved with every embodiment.

Various aspects are described in this disclosure, which define the invention only insofar as they correspond to the claims, and which include, but are not limited to, the following aspects:
1. A medication delivery device comprising:
   a housing comprising a reservoir sized sufficiently to hold medication;
   a dose button being rotatable relative to the housing to select a dose size of the medication for an injection;
   a printed circuit board;
   a conductive trace disposed at least partially on the printed circuit board;
   a bias source in electrical communication with the conductive trace; and
   a microcontroller in electrical communication with the conductive trace through a logic input to the microcontroller, the microcontroller being configured to:
   receive a signal from the conductive trace through the logic input; and
   determine, based on the received signal, that moisture may be present on the printed circuit board.
2. The medication delivery device of aspect 1, wherein the logic input is a general-purpose input/output (GPIO) to the microcontroller.
3. The medication delivery device of any of aspects 1-2, further comprising a switch mounted to the printed circuit board, wherein the microcontroller is configured to:
   receive a set of signals from the switch;
   generate, based on the set of signals, a count of the set of signals.
4. The medication delivery device of aspect 3, wherein the medication delivery device further comprising a rotatable element that is rotatable relative to the printed circuit board, the rotatable element having a series of protrusions that are spaced from one another, the rotatable element being positioned to permit the protrusions to slide against the switch to move the switch between a closed position and an open position as the rotatable element rotates.
5. The medication delivery device of aspect 3, wherein the switch comprises a piezoelectric sensor, a magnetic sensor, an accelerometer, an optical sensor, an interrupter sensor, an optical encoder, or some combination thereof.
6. The medication delivery device of any of aspects 3-5, wherein the microcontroller is further configured to, upon determining that moisture may be present on the printed circuit board:
   discard the generated count of the set of signals, such that the generated count is (a) not stored in a memory in communication with the microcontroller, (b) not transmitted to a remote device through a communication module in communication with the microcontroller, or some combination thereof.
7. The medication delivery device of any of aspects 1-6, wherein the microcontroller is further configured to, upon determining that moisture may be present on the printed circuit board:
   change an operating state of the microcontroller, such that the microcontroller (a) enters a hibernation state, (b) powers down, (c) restarts, or some combination thereof.
8. The medication delivery device of any of aspects 1-7, wherein the microcontroller is further configured to, upon determining that moisture may be present on the printed circuit board:
   transmit, via a communication module in communication with the microcontroller, at least one message comprising data indicative of the determination of possible moisture on the printed circuit board.
9. The medication delivery device of aspect 8, wherein the at least one message is configured to cause an external mobile device in communication with the medication delivery device to discard or disregard one or more previously transmitted count values.
10. The medication delivery device of any of aspects 1-9, wherein the bias source comprises a resistor disposed as part of a System on Chip (SoC) that includes the microcontroller.
11. The medication delivery device of any of aspects 1-9, wherein the bias source comprises a resistor disposed at least partially on the printed circuit board and external to the SoC that includes the microcontroller.
12. The medication delivery device of any of aspects 1-9, wherein the bias source comprises at least one of a diode, a transistor, and a capacitor.
13. The medication delivery device of any of aspects 1-12, wherein:
   the bias source is configured to provide approximately 0 volts to the conductive trace, such that the microcontroller reads a logical 0 from the logic input; and
   receiving the signal comprises reading a logical 1 from the logic input; and
   determining, based on the received signal, that moisture may be present on the printed circuit board comprises determining the logic input changed from a logical 0 to a logical 1.
14. The medication delivery device of any of aspects 1-12, wherein:
   the bias source is configured to provide a positive voltage to the conductive trace, such that the microcontroller reads a logical 1 from the logic input; and
   receiving the signal from the conductive trace through the logic input comprises reading a logical 0 from the logic input; and
   determining, based on the received signal, that moisture may be present on the printed circuit board comprises determining the logic input changed from a logical 1 to a logical 0.
15. The medication delivery device of any of aspects 1-13, wherein the device further comprises medication held within the reservoir.
16. The medication delivery device of aspect 15, wherein the medication is an insulin.
17. The medication delivery device of any of aspects 1-16, wherein the conductive trace is in a plane of the printed circuit board.
18. The medication delivery device of any of aspects 1-17, wherein the conductive trace is an exposed test pad.
19. A computerized method for execution by a microcontroller of a medication delivery device, wherein:
   the medication delivery device comprises:
   a housing comprising a reservoir sized sufficiently to hold medication; and
   a dose button being rotatable relative to the housing to select a dose size of the medication for an injection; and
   the microcontroller is in electrical communication with, through a logic input to the microcontroller, a conductive trace disposed at least partially on a printed circuit board;
   the method comprising:
      receiving a signal from the conductive trace through the logic input; and
      determining, based on the received signal, that moisture may be present on the printed circuit board.
20. A medication delivery device comprising:
   a housing comprising a reservoir sized sufficiently to hold medication;
   a dose button being rotatable relative to the housing to select a dose size of the medication for an injection;
   a printed circuit board;
   a conductive trace disposed at least partially on the printed circuit board;
   an analog-to-digital converter (ADC) comprising an input and an output, wherein:
   the input of the ADC is in electrical communication with the conductive trace; and
   an operating input range of the ADC is between a low input voltage and a high input voltage;
   a bias source in electrical communication with the input of the ADC, wherein the bias source is configured to provide a bias voltage between the low input voltage and the high input voltage; and
   a microcontroller in electrical communication with the output of the ADC, the microcontroller being configured to:
      receive a signal from the output of the ADC; and
      determine, based on the received signal, that moisture may be present on the printed circuit board.
21. The medication delivery device of aspect 20, wherein:
   the low input voltage of the operating input range of the ADC is a ground voltage; and
   the high input voltage of the operating input range of the ADC is a positive voltage.
22. The medication delivery device of aspect 21, wherein the ground voltage is approximately 0 volts and the positive voltage is approximately 0.9 volts.
23. The medication delivery device of any of aspects 20-22, wherein the bias voltage provided by the bias source is between 25% and 75% of the operating input range of the ADC.
24. The medication delivery device of any of aspects 20-22, wherein the bias voltage provided by the bias source is 40% and 60% of the operating input range of the ADC.
25. The medication delivery device of any of aspects 20-24, wherein the bias voltage is 50% of the operating voltage range of the ADC.
26. The medication delivery device of any of aspects 20-25, wherein the bias source comprises a capacitive coupling between the input of the ADC and a power source.
27. The medication delivery device of any of aspects 20-25, wherein the bias source comprises:
   a first resistor connected to circuitry configured to provide approximately the low input voltage; and
   a second resistor connected to circuitry configured to provide approximately the high input voltage.
28. The medication delivery device of any of aspects 20-25, wherein the bias source comprises at least one of a diode, a transistor, and a capacitor.
29. The medication delivery device of any of aspects 20-28, wherein:
   receiving the signal comprises reading a voltage from the output of the ADC; and
   determining, based on the received signal, that moisture may be present on the printed circuit board comprises determining the received voltage is greater than a preset threshold voltage amount different than the bias voltage.
30. The medication delivery device of any of aspects 20-29, wherein the device further comprises medication held within the reservoir.
31. The medication delivery device of aspect 30, wherein the medication is an insulin.
32. A computerized method being executed by a microcontroller of a medication delivery device, wherein:
   the medication delivery device comprises:
   a housing comprising a reservoir sized sufficiently to hold medication; and
   a dose button being rotatable relative to the housing to select a dose size of the medication for an injection; and
   the microcontroller is in electrical communication with an output of an analog-to-digital converter (ADC), wherein:
      an operating input range of the ADC is between a low input voltage and a high input voltage; and
      an input of the ADC is in electrical communication with (a) a conductive trace disposed at least partially on a printed circuit board, and (b) a bias source, wherein the bias source is configured to provide a bias voltage between the low input voltage and the high input voltage; and
      the method comprising:
         receiving a signal from the output of the ADC; and
         determining, based on the received signal, that moisture may be present on the printed circuit board.

## Claims

1. A medication delivery device (10) comprising:
a housing (12) comprising a reservoir (20) sized sufficiently to hold medication;
a dose button (30) being rotatable relative to the housing (12) to select a dose size of the medication for an injection; and
a printed circuit board (2100);
**characterized in that** the medication delivery device (10) comprises a conductive trace (2114) disposed at least partially on the printed circuit board (2100);
a bias source (2102) in electrical communication with the conductive trace (2114); and
a microcontroller (2104) in electrical communication with the conductive trace (2114) through a logic input to the microcontroller (2104) such that, in the absence of moisture, the bias source (2102) holds the logic input to the microcontroller (2104) at an expected voltage, the microcontroller (2104) being configured to:
receive a signal from the conductive trace (2114) through the logic input; and
determine, based on the received signal, that moisture may be present on the printed circuit board (2100) if the logic input deviates from the expected voltage by greater than a threshold amount.

2. The medication delivery device (10) of claim 1, wherein the logic input is a general-purpose input/output, GPIO, (2108) to the microcontroller (2104).

3. The medication delivery device (10) of any of claims 1-2, further comprising a switch (2110) mounted to the printed circuit board (2100), wherein the microcontroller (2104) is configured to:
receive a set of signals from the switch (2110);
generate, based on the set of signals, a count of the set of signals.

4. The medication delivery device (10) of claim 3, wherein the medication delivery device (10) further comprising a rotatable element that is rotatable relative to the printed circuit board (2100), the rotatable element having a series of protrusions that are spaced from one another, the rotatable element being positioned to permit the protrusions to slide against the switch (2110) to move the switch (2110) between a closed position and an open position as the rotatable element rotates.

5. The medication delivery device (10) of claim 3, wherein the switch (2110) comprises a piezoelectric sensor, a magnetic sensor, an accelerometer, an optical sensor, an interrupter sensor, an optical encoder, or some combination thereof.

6. The medication delivery device (10) of any of claims 3-5, wherein the microcontroller (2104) is further configured to, upon determining that moisture may be present on the printed circuit board (2100):
discard the generated count of the set of signals, such that the generated count is (a) not stored in a memory in communication with the microcontroller (2104), (b) not transmitted to a remote device through a communication module in communication with the microcontroller (2104), or some combination thereof.

7. The medication delivery device (10) of any of claims 1-6, wherein the microcontroller (2104) is further configured to, upon determining that moisture may be present on the printed circuit board (2100):
change an operating state of the microcontroller (2104), such that the microcontroller (2104) (a) enters a hibernation state, (b) powers down, (c) restarts, or some combination thereof.

8. The medication delivery device (10) of any of claims 1-7, wherein the microcontroller (2104) is further configured to, upon determining that moisture may be present on the printed circuit board (2100):
transmit, via a communication module in communication with the microcontroller (2104), at least one message comprising data indicative of the determination of possible moisture on the printed circuit board (2100).

9. The medication delivery device (10) of claim 8, wherein the at least one message is configured to cause an external mobile device in communication with the medication delivery device (10) to discard or disregard one or more previously transmitted count values.

10. The medication delivery device (10) of any of claims 1-9, wherein the bias source (2102) comprises:
a resistor disposed as part of a System on Chip, SoC, (2116) that includes the microcontroller (2104);
a resistor disposed at least partially on the printed circuit board (2100) and external to the SoC (2116) that includes the microcontroller (2104); or
at least one of a diode, a transistor, and a capacitor.

11. The medication delivery device (10) of any of claims 1-10, wherein:
the bias source (2102) is configured to provide approximately 0 volts to the conductive trace (2114), such that the microcontroller (2104) reads a logical 0 from the logic input; and
receiving the signal comprises reading a logical 1 from the logic input; and
determining, based on the received signal, that moisture may be present on the printed circuit board (2100) comprises determining the logic input changed from a logical 0 to a logical 1.

12. The medication delivery device (10) of any of claims 1-10, wherein:
the bias source (2102) is configured to provide a positive voltage to the conductive trace (2114), such that the microcontroller (2104) reads a logical 1 from the logic input; and
receiving the signal from the conductive trace (2114) through the logic input comprises reading a logical 0 from the logic input; and
determining, based on the received signal, that moisture may be present on the printed circuit board (2100) comprises determining the logic input changed from a logical 1 to a logical 0.

13. The medication delivery device (10) of any of claims 1-11, wherein the device (10) further comprises medication held within the reservoir (20), wherein, optionally, the medication is an insulin.

14. The medication delivery device (10) of any of claims 1-13, wherein the conductive trace (2114) is:
in a plane of the printed circuit board (2100); and/or
an exposed test pad.

15. A computerized method for execution by a microcontroller (2104) of a medication delivery device (10), wherein:
the medication delivery device (10) comprises:
a housing (12) comprising a reservoir (20) sized sufficiently to hold medication; and
a dose button (30) being rotatable relative to the housing (12) to select a dose size of the medication for an injection, **characterized in that**:
the microcontroller (2104) is in electrical communication with, through a logic input to the microcontroller (2104), a conductive trace (2114) disposed at least partially on a printed circuit board (2100), wherein the conductive trace (2114) is in electrical communication with a bias source (2102) that, in the absence of moisture, holds the logic input to the microcontroller (2104) at an expected voltage;
the method comprising:
receiving a signal from the conductive trace (2114) through the logic input; and
determining, based on the received signal, that moisture may be present on the printed circuit board (2100) if the logic input deviates from the expected voltage by greater than a threshold amount.

## Patentansprüche

1. Medikamentenabgabevorrichtung (10), umfassend:
ein Gehäuse (12), umfassend ein Reservoir (20), das ausreichend dimensioniert ist, um ein Medikament aufzunehmen;
einen Dosisknopf (30), der relativ zu dem Gehäuse (12) drehbar ist, um eine Dosisgröße des Medikaments für eine Injektion auszuwählen; und
eine Leiterplatte (2100);
**dadurch gekennzeichnet, dass** die Medikamentenabgabevorrichtung (10) eine leitfähige Spur (2114) umfasst, die mindestens teilweise auf der Leiterplatte (2100) angeordnet ist;
eine Vorspannungsquelle (2102) in elektrischer Kommunikation mit der leitfähigen Spur (2114); und
einen Mikrocontroller (2104) in elektrischer Kommunikation mit der leitfähigen Spur (2114) über einen Logikeingang an den Mikrocontroller (2104), derart, dass in der Abwesenheit von Feuchtigkeit die Vorspannungsquelle (2102) den Logikeingang an den Mikrocontroller (2104) auf einer erwarteten Spannung hält, wobei der Mikrocontroller (2104) konfiguriert ist zum:
Empfangen eines Signals von der leitfähigen Spur (2114) über den Logikeingang; und
Bestimmen, basierend auf dem empfangenen Signal, dass Feuchtigkeit auf der Leiterplatte (2100) vorhanden sein kann, falls der Logikeingang um mehr als eine Schwellenmenge von der erwarteten Spannung abweicht.

2. Medikamentenabgabevorrichtung (10) nach Anspruch 1, wobei der Logikeingang ein Allzweck-Eingang/Ausgang, GPIO, (2108) an den Mikrocontroller (2104) ist.

3. Medikamentenabgabevorrichtung (10) nach einem der Ansprüche 1 bis 2, ferner umfassend einen Schalter (2110), der an der Leiterplatte (2100) montiert ist, wobei der Mikrocontroller (2104) konfiguriert ist zum:
Empfangen eines Satzes von Signalen von dem Schalter (2110);
Erzeugen, basierend auf dem Satz von Signalen, einer Zählung des Satzes von Signalen.

4. Medikamentenabgabevorrichtung (10) nach Anspruch 3, die Medikamentenabgabevorrichtung (10) ferner umfassend ein drehbares Element, das relativ zu der Leiterplatte (2100) drehbar ist, wobei das drehbare Element eine Reihe von Vorsprüngen aufweist, die voneinander beabstandet sind, wobei das drehbare Element positioniert ist, um den Vorsprüngen zu ermöglichen, gegen den Schalter (2110) zu gleiten, um den Schalter (2110) zwischen einer geschlossenen Position und einer offenen Position zu bewegen, während sich das drehbare Element dreht.

5. Medikamentenabgabevorrichtung (10) nach Anspruch 3, wobei der Schalter (2110) einen piezoelektrischen Sensor, einen magnetischen Sensor, einen Beschleunigungssensor, einen optischen Sensor, einen Unterbrechersensor, einen optischen Codierer oder eine Kombination davon umfasst.

6. Medikamentenabgabevorrichtung (10) nach einem der Ansprüche 3 bis 5, wobei der Mikrocontroller (2104) ferner, bei dem Bestimmen, dass Feuchtigkeit auf der Leiterplatte (2100) vorhanden sein kann, konfiguriert ist zum:
derartigen Verwerfen der erzeugten Zählung des Satzes von Signalen, dass die erzeugte Zählung (a) nicht in einem Speicher in Kommunikation mit dem Mikrocontroller (2104) gespeichert wird, (b) nicht über ein Kommunikationsmodul in Kommunikation mit dem Mikrocontroller (2104) an eine entfernte Vorrichtung übertragen wird, oder eine Kombination davon.

7. Medikamentenabgabevorrichtung (10) nach einem der Ansprüche 1 bis 6, wobei der Mikrocontroller (2104) ferner, bei dem Bestimmen, dass Feuchtigkeit auf der Leiterplatte (2100) vorhanden sein kann, konfiguriert ist zum:
derartigen Ändern eines Betriebszustands des Mikrocontrollers (2104), dass der Mikrocontroller (2104) (a) in einen Ruhezustand übergeht, (b) abschaltet, (c) neu startet oder eine Kombination davon.

8. Medikamentenabgabevorrichtung (10) nach einem der Ansprüche 1 bis 7, wobei der Mikrocontroller (2104) ferner, bei dem Bestimmen, dass Feuchtigkeit auf der Leiterplatte (2100) vorhanden sein kann, konfiguriert ist zum:
Übertragen, über ein Kommunikationsmodul, das mit dem Mikrocontroller (2104) in Kommunikation steht, mindestens einer Nachricht, umfassend Daten, die auf die Bestimmung möglicher Feuchtigkeit auf der Leiterplatte (2100) hinweisen.

9. Medikamentenabgabevorrichtung (10) nach Anspruch 8, wobei die mindestens eine Nachricht konfiguriert ist, um eine externe mobile Vorrichtung, die mit der Medikamentenabgabevorrichtung (10) in Kommunikation steht, zu veranlassen, einen oder mehrere zuvor übertragene Zählwerte zu verwerfen oder unberücksichtigt zu lassen.

10. Medikamentenabgabevorrichtung (10) nach einem der Ansprüche 1 bis 9, wobei die Vorspannungsquelle (2102) umfasst:
einen Widerstand, der als Teil eines System-on-Chip, SoC, (2116) angeordnet ist, das den Mikrocontroller (2104) einschließt;
einen Widerstand, der mindestens teilweise auf der Leiterplatte (2100) angeordnet ist und sich außerhalb des SoC (2116) befindet, das den Mikrocontroller (2104) einschließt; oder
mindestens eines von einer Diode, einem Transistor und einem Kondensator.

11. Medikamentenabgabevorrichtung (10) nach einem der Ansprüche 1 bis 10, wobei:
die Vorspannungsquelle (2102) konfiguriert ist, um der leitfähigen Spur (2114) etwa 0 Volt derart bereitzustellen, dass der Mikrocontroller (2104) eine logische 0 von dem Logikeingang liest; und
das Empfangen des Signals das Lesen einer logischen 1 von dem Logikeingang umfasst; und
das Bestimmen basierend auf dem empfangenen Signal, dass Feuchtigkeit auf der Leiterplatte (2100) vorhanden sein kann, das Bestimmen umfasst, dass sich der Logikeingang von einer logischen 0 auf eine logische 1 geändert hat.

12. Medikamentenabgabevorrichtung (10) nach einem der Ansprüche 1 bis 10, wobei:
die Vorspannungsquelle (2102) konfiguriert ist, um der leitfähigen Spur (2114) eine positive Spannung derart bereitzustellen, dass der Mikrocontroller (2104) eine logische 1 von dem Logikeingang liest; und
das Empfangen des Signals von der leitfähigen Spur (2114) über den Logikeingang das Lesen einer logischen 0 von dem Logikeingang umfasst; und
das Bestimmen, basierend auf dem empfangenen Signal, dass Feuchtigkeit auf der Leiterplatte (2100) vorhanden sein kann, das Bestimmen umfasst, dass sich der Logikeingang von einer logischen 1 auf eine logische 0 geändert hat.

13. Medikamentenabgabevorrichtung (10) nach einem der Ansprüche 1 bis 11, wobei die Vorrichtung (10) ferner ein in dem Reservoir (20) gehaltenes Medikament umfasst, wobei das Medikament optional ein Insulin ist.

14. Medikamentenabgabevorrichtung (10) nach einem der Ansprüche 1 bis 13, wobei die leitfähige Spur (2114) ist:
in einer Ebene der Leiterplatte (2100); und/oder
ein freiliegendes Testpad.

15. Computergestütztes Verfahren für die Ausführung durch einen Mikrocontroller (2104) einer Medikamentenabgabevorrichtung (10), wobei:
die Medikamentenabgabevorrichtung (10) umfasst:
ein Gehäuse (12), umfassend ein Reservoir (20), das ausreichend dimensioniert ist, um ein Medikament aufzunehmen; und
ein Dosisknopf (30), der relativ zu dem Gehäuse (12) drehbar ist, um eine Dosisgröße des Medikaments für eine Injektion auszuwählen,
**dadurch gekennzeichnet, dass:**
der Mikrocontroller (2104) in elektrischer Kommunikation mit, über einen Logikeingang an den Mikrocontroller (2104), einer leitfähigen Spur (2114) steht, die mindestens teilweise auf einer Leiterplatte (2100) angeordnet ist, wobei die leitfähige Spur (2114) in elektrischer Kommunikation mit einer Vorspannungsquelle (2102) steht, die in der Abwesenheit von Feuchtigkeit den Logikeingang an den Mikrocontroller (2104) auf einer erwarteten Spannung hält;
das Verfahren umfassend:
Empfangen eines Signals von der leitfähigen Spur (2114) durch den Logikeingang;
und
Bestimmen, basierend auf dem empfangenen Signal, dass Feuchtigkeit auf der Leiterplatte (2100) vorhanden sein kann, falls der Logikeingang um mehr als eine Schwellenmenge von der erwarteten Spannung abweicht.

## Revendications

1. Dispositif de délivrance de médicament (10) comprenant :
un logement (12) comprenant un réservoir (20) dimensionné de manière suffisante pour conserver un médicament ;
un bouton de dose (30) étant rotatif par rapport au logement (12) pour sélectionner une taille de dose du médicament pour une injection ; et
une carte à circuit imprimé (2100) ;
**caractérisé en ce que** le dispositif de délivrance de médicament (10) comprend un tracé conducteur (2114) disposé au moins partiellement sur la carte à circuit imprimé (2100) ;
une source de polarisation (2102) en communication électrique avec le tracé conducteur (2114) ; et
un microcontrôleur (2104) en communication électrique avec le tracé conducteur (2114) par le biais d'une entrée logique vers le microcontrôleur (2104) de telle sorte que, en l'absence d'humidité, la source de polarisation (2102) maintient l'entrée logique vers le microcontrôleur (2104) à une tension attendue, le microcontrôleur (2104) étant configuré pour :
recevoir un signal provenant du tracé conducteur (2114) par le biais de l'entrée logique ; et
déterminer, en fonction du signal reçu, que de l'humidité peut être présente sur la carte à circuit imprimé (2100) si l'entrée logique s'écarte de la tension attendue de plus d'une quantité seuil.

2. Dispositif de délivrance de médicament (10) selon la revendication 1, dans lequel l'entrée logique est une entrée/sortie d'usage général, GPIO, (2108) vers le microcontrôleur (2104).

3. Dispositif de délivrance de médicament (10) selon l'une quelconque des revendications 1 à 2, comprenant en outre un commutateur (2110) monté sur la carte à circuit imprimé (2100), dans lequel le microcontrôleur (2104) est configuré pour :
recevoir un ensemble de signaux provenant du commutateur (2110) ;
générer, en fonction de l'ensemble de signaux, un comptage de l'ensemble de signaux.

4. Dispositif de délivrance de médicament (10) selon la revendication 3, dans lequel le dispositif de délivrance de médicament (10) comprend en outre un élément rotatif qui est rotatif par rapport à la carte à circuit imprimé (2100), l'élément rotatif ayant une série de parties saillantes qui sont espacées les unes des autres, l'élément rotatif étant positionné pour permettre aux parties saillantes de coulisser contre le commutateur (2110) pour déplacer le commutateur (2110) entre une position fermée et une position ouverte à mesure que l'élément rotatif effectue une rotation.

5. Dispositif de délivrance de médicament (10) selon la revendication 3, dans lequel le commutateur (2110) comprend un capteur piézoélectrique, un capteur magnétique, un accéléromètre, un capteur optique, un capteur interrupteur, un encodeur optique, ou une certaine combinaison de ceux-ci.

6. Dispositif de délivrance de médicament (10) selon l'une quelconque des revendications 3 à 5, dans lequel le microcontrôleur (2104) est configuré en outre, lorsqu'on détermine que de l'humidité peut être présente sur la carte à circuit imprimé (2100) pour :
écarter le comptage généré de l'ensemble de signaux, de telle sorte que le comptage généré (a) n'est pas stocké dans une mémoire en communication avec le microcontrôleur (2104), (b) n'est pas transmis à un dispositif distant par le biais d'un module de communication en communication avec le microcontrôleur (2104), ou une certaine combinaison de ceux-ci.

7. Dispositif de délivrance de médicament (10) selon l'une quelconque des revendications 1 à 6, dans lequel le microcontrôleur (2104) est configuré en outre, lorsqu'on détermine que de l'humidité peut être présente sur la carte à circuit imprimé (2100), pour :
changer un état de fonctionnement du microcontrôleur (2104), de telle sorte que le microcontrôleur (2104) (a) entre dans un état de veille prolongée, (b) se met hors tension, (c) redémarre, ou une certaine combinaison de ceux-ci.

8. Dispositif de délivrance de médicament (10) selon l'une quelconque des revendications 1 à 7, dans lequel le microcontrôleur (2104) est configuré en outre, lorsqu'on détermine que de l'humidité peut être présente sur la carte à circuit imprimé (2100), pour :
transmettre, par l'intermédiaire d'un module de communication en communication avec le microcontrôleur (2104), au moins un message comprenant des données indiquant la détermination d'humidité possible sur la carte à circuit imprimé (2100).

9. Dispositif de délivrance de médicament (10) selon la revendication 8, dans lequel l'au moins un message est configuré pour amener un dispositif mobile externe en communication avec le dispositif de délivrance de médicament (10) à écarter ou à ignorer une ou plusieurs valeurs de comptage précédemment transmises.

10. Dispositif de délivrance de médicament (10) selon l'une quelconque des revendications 1 à 9, dans lequel la source de polarisation (2102) comprend :
une résistance disposée dans le cadre d'une puce-système, SoC, (2116) qui comporte le microcontrôleur (2104) ;
une résistance disposée au moins partiellement sur la carte à circuit imprimé (2100) et externe à la SoC (2116) qui comporte le
microcontrôleur (2104) ; ou
au moins l'un parmi une diode, un transistor et un condensateur.

11. Dispositif de délivrance de médicament (10) selon l'une quelconque des revendications 1 à 10, dans lequel :
la source de polarisation (2102) est configurée pour fournir approximativement 0 volt au tracé conducteur (2114), de telle sorte que le microcontrôleur (2104) lit un 0 logique à partir de l'entrée logique ; et
la réception du signal comprend la lecture d'un 1 logique à partir de l'entrée logique ; et
la détermination, en fonction du signal reçu, que de l'humidité peut être présente sur la carte à circuit imprimé (2100) comprend la détermination que l'entrée logique a changé d'un 0 logique à un 1 logique.

12. Dispositif de délivrance de médicament (10) selon l'une quelconque des revendications 1 à 10, dans lequel :
la source de polarisation (2102) est configurée pour fournir une tension positive au tracé conducteur (2114), de telle sorte que le microcontrôleur (2104) lit un 1 logique à partir de l'entrée logique ; et
la réception du signal provenant du tracé conducteur (2114) par le biais de l'entrée logique comprend la lecture d'un 0 logique à partir de l'entrée logique ; et
la détermination, en fonction du signal reçu, que de l'humidité peut être présente sur la carte à circuit imprimé (2100) comprend la détermination que l'entrée logique a changé d'un 1 logique à un 0 logique.

13. Dispositif de délivrance de médicament (10) selon l'une quelconque des revendications 1 à 11, dans lequel le dispositif (10) comprend en outre un médicament conservé au sein du réservoir (20), dans lequel, facultativement, le médicament est une insuline.

14. Dispositif de délivrance de médicament (10) selon l'une quelconque des revendications 1 à 13, dans lequel le tracé conducteur (2114) est :
dans un plan de la carte à circuit imprimé (2100) ; et/ou
un tampon de test exposé.

15. Procédé informatisé pour exécution par un microcontrôleur (2104) d'un dispositif de délivrance de médicament (10), dans lequel :
le dispositif de délivrance de médicament (10) comprend :
un logement (12) comprenant un réservoir (20) dimensionné de manière suffisante pour conserver un médicament ; et
un bouton de dose (30) étant rotatif par rapport au logement (12) pour sélectionner une taille de dose du médicament pour une injection,
**caractérisé en ce que** :
le microcontrôleur (2104) est en communication électrique, par le biais d'une entrée logique vers le microcontrôleur (2104), avec un tracé conducteur (2114) disposé au moins partiellement sur une carte à circuit imprimé (2100), dans lequel le tracé conducteur (2114) est en communication électrique avec une source de polarisation (2102) qui, en l'absence d'humidité, maintient l'entrée logique vers le microcontrôleur (2104) à une tension attendue ;
le procédé comprenant :
la réception d'un signal provenant du tracé conducteur (2114) par le biais de l'entrée
logique ; et
la détermination, en fonction du signal reçu, que de l'humidité peut être présente sur la carte à circuit imprimé (2100) si l'entrée logique s'écarte de la tension attendue de plus d'une quantité seuil.
